# EUROPEAN PATENT APPLICATION

(11) **EP 2 998 396 A1**
(43) Date of publication of application: **23.03.2016**
(21) Application number: 14185019.8
(22) Date of filing: 16.09.2014
(51) Int. Cl.: C12N 15/01, C07K 14/705, A61K 31/713, C12N 15/11, C12N 15/113, G01N 33/574

(54) **Manipulation of hairy and enhancer of split 3 (Hes3) and its regulators/mediators in diabetes, obesity, and metabolic syndrome**

(71) Applicant: Technische Universität Dresden, 01062 Dresden (DE)
(72) Inventor: Androutsellis-Theotokis, Andreas, 01307 Dresden (DE); Nikolakopoulou, Polyxeni, 01099 Dresden (DE); Masjkur, Jimmy, 01307 Dresden (DE); Poser, Steven Walter, 01307 Dresden (DE)
(74) Representative: Vossius & Partner Patentanwälte Rechtsanwälte mbB

(57) **Abstract**

The present invention relates to an inhibitor of Hairy and Enhancer of Split 3 (Hes3) for use in a method of preventing or treating (i) a disease characterised by insulin resistance or (ii) obesity. Further, the present invention also relates to a method of preventing or treating a disease characterised by insulin resistance or obesity, to a pharmaceutical composition comprising Hes3 and to Hes3 and/or an activator thereof for use in a method of preventing or treating a disease characterised by decreased insulin production. In addition, the present invention relates to a method of preventing or treating a disease characterised by decreased insulin production and to the use of Hes3 as a target in a screen for identifying a compound suitable as a lead compound and/or as a medicament for the treatment of diseases characterised by insulin resistance or of obesity or of diseases characterised by decreased insulin production. Moreover, the present invention relates to *in-vitro* or *ex-vivo* methods for identifying a compound suitable as a lead compound and/or as a medicament for the treatment and/or prevention of diseases characterised by insulin resistance or of obesity or of diseases characterised by decreased insulin production. Finally, the present invention also relates to an in-vitro method of eliciting or enhancing insulin production.

## Description

The present invention relates to an inhibitor of Hairy and Enhancer of Split 3 (Hes3) for use in a method of preventing or treating (i) a disease characterised by insulin resistance or (ii) obesity. Further, the present invention also relates to a method of preventing or treating a disease characterised by insulin resistance or obesity, to a pharmaceutical composition comprising Hes3 and to Hes3 and/or an activator thereof for use in a method of preventing or treating a disease characterised by decreased insulin production. In addition, the present invention relates to a method of preventing or treating a disease characterised by decreased insulin production and to the use of Hes3 as a target in a screen for identifying a compound suitable as a lead compound and/or as a medicament for the treatment of diseases characterised by insulin resistance or of obesity or of diseases characterised by decreased insulin production. Moreover, the present invention relates to *in-vitro* or *ex-vivo* methods for identifying a compound suitable as a lead compound and/or as a medicament for the treatment and/or prevention of diseases characterised by insulin resistance or of obesity or of diseases characterised by decreased insulin production. Finally, the present invention also relates to an *in-vitro* method of eliciting or enhancing insulin production.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Insulin is the only blood glucose-lowering hormone and is secreted by the pancreatic beta-cells of the islets of Langerhans. After its synthesis, the hormone is processed into its biologically active form and stored in vesicles ready for release. In the event insulin secretion becomes necessary, the hormone is released via a complex process involving the interplay of several proteins with the vesicle (Rorsman and Renström (2003) Diabetologia 46, 1029).

Thus, pancreatic islets are strictly required for maintaining glucose homeostasis, and defects in their ability to adequately secrete insulin in response to glucose result in diabetes (Bell and Polonsky (2001) Nature 414, 788).

Diabetes is a chronic disease, which occurs when the pancreas does not produce enough insulin, or when the body cannot effectively use the insulin it produces (insulin resistance). According to the WHO, 347 million people worldwide have diabetes (Danaei et al. (2011) Lancet, 378(9785):31-40). In 2004, an estimated 3.4 million people died from consequences of high fasting blood sugar (Global health risks. Mortality and burden of disease attributable to selected major risks. Geneva, World Health Organization, 2009) and the WHO projected that diabetes will be the 7th leading cause of death in 2030 (Global status report on noncommunicable diseases 2010. Geneva, World Health Organization, 2011).

Diabetes can be divided into different subtypes which differ in the mechanism underlying the disease.

Type 1 diabetes (previously known as insulin-dependent, juvenile or childhood-onset) is characterised by deficient insulin production which is considered to be due to autoimmune destruction of the insulin-producing beta cells. The cause of type 1 diabetes is not known and it is not preventable with current knowledge. Symptoms, which may occur suddenly, include excessive excretion of urine (polyuria), thirst (polydipsia), constant hunger, weight loss, vision changes and fatigue. Type 1 diabetes is usually treated with insulin, exercise, and a diabetic diet.

Type 2 diabetes (formerly called non-insulin-dependent or adult-onset) results from the body's ineffective use of insulin also referred to as insulin resistance. Insulin resistance occurs when cells in the body such as, for example, liver, skeletal muscle and adipose/fat tissue become less sensitive and eventually resistant to insulin, the hormone which is produced by the pancreas to facilitate glucose absorption. Glucose can no longer be absorbed by the cells but remains in the blood, triggering the need for more and more insulin (hyperinsulinaemia) to be produced in an attempt to process the glucose. The production of ever-increasing amounts of insulin strains and may eventually damage the beta cells in the pancreas. Once the pancreas is no longer able to produce enough insulin, the concentration of glucose in the blood increases (hyperglycaemia). This leads to damage to the body including a build-up of triglycerides which further impairs insulin sensitivity and causes damage to the body's microvascular system (leading to kidney, eye and nerve damage).

Thus, insulin resistance is strongly associated with irregularities in both glucose and lipid metabolism, and is therefore an underlying feature of the metabolic syndrome and type 2 diabetes. Approximately 90% of people with diabetes around the world are suffering from type 2 diabetes (Definition, diagnosis and classification of diabetes mellitus and its complications. Part 1: Diagnosis and classification of diabetes mellitus. Geneva, World Health Organization, 1999 (WHO/NCD/NCS/99.2)). This type of diabetes is considered to largely be the result of excess body weight and physical inactivity. Symptoms may be similar to those of type 1 diabetes, but are often less marked. As a result, the disease may be diagnosed not earlier than several years after onset, once complications have already arisen. Treatment for type 2 diabetes includes weight reduction, exercise, a diabetic diet, oral medication where necessary and if this is not sufficient, treatment with insulin. Medications for type 2 diabetes are designed to increase the insulin output by the pancreas, decrease the amount of glucose released from the liver, increase the sensitivity of cells to insulin, decrease the absorption of carbohydrates from the intestine, and/or slow emptying of the stomach to delay the presentation of carbohydrates for digestion and absorption in the small intestine. Medications increasing the release of insulin from the pancreas include sulfonylureas, such as for example chlorpropamide, tolbutamide, glyburide, glipizide and glimepiride, and meglitinides. These drugs are effective in rapidly lowering blood sugar but run the risk of causing hypoglycemia. In addition, in some patients allergic reactions may prevent use of sulfonylureas.

Biguanides such as metformin decrease glucose production by the liver. Because insulin production is not directly affected, hypoglycaemia is usually not an issue with these drugs. However, their use is limited in patients with kidney or liver impairment.

As a further alternative, sodium-glucose co-transporter 2 (SGLT2) inhibitors such as canagliflozin may be employed to block reabsorption of glucose by the kidney and to thereby lower the blood glucose levels. The class of drugs known as thiazolidinediones lowers blood glucose by improving target cell response to insulin, i.e. by decreasing insulin resistance. However, some substances falling within this class have been associated with liver toxicity or with increased risk of heart attack and stroke.

Further, alpha glucosidase inhibitors can be used to decrease breakdown of carbohydrates in the small intestine and to thereby delay glucose absorption. In addition, amylin analogues, incretin mimetics, glucagon-like peptide-1 (GLP-1) agonists, dipeptidyl peptidase (DPP-IV) inhibitors and combination therapies are also used in the treatment of type 2 diabetes.

A further subform of diabetes is gestational diabetes. Gestational diabetes is a type of diabetes wherein hyperglycaemia first occurs or is first recognised during pregnancy. Gestational diabetes is most often diagnosed through prenatal screening, rather than reported symptoms. In many cases gestational diabetes is a transient and reversible form of diabetes. Treatment options include exercise and a diabetic diet as well as insulin where required.

A complex of several interrelated risk factors for diabetes as well as for cardiovascular disease (CVD) and other diseases is referred to as metabolic syndrome. Alternative terms to describe metabolic syndrome are, for example, "syndrome X", "Reaven's syndrome" and in Australia, "CHAOS". The factors of this syndrome include dysglycemia, raised blood pressure, elevated triglyceride levels, low high-density lipoprotein cholesterol levels, and obesity (particularly central adiposity). Most commonly, patients suffering from metabolic syndrome exhibit abdominal obesity and resistance to insulin.

According to the International Diabetes Federation (IDF), a quarter of the world's adults have metabolic syndrome and people with metabolic syndrome have a five-fold greater risk of developing type 2 diabetes (Alberti et al., (2009) Circulation 120: 1640-1645).

Treatment of metabolic syndrome is directed at lowering LDL cholesterol and high blood pressure and preventing or managing diabetes. As a first line of treatment losing weight, adhering to a healthy diet, exercising and quitting smoking (where applicable) are recommended. In addition, treatment of the individual factors may be required. For example, elevated cholesterol levels are treated with medicines such as statins, fibrates, or nicotinic acid. High blood pressure is treated, for example, with diuretics or ACE inhibitor and high blood sugar is treated with oral medicines (such as metformin), insulin injections, or both. Moreover, low-dose aspirin can help reduce the risk of blood clots, especially for people whose risk of heart disease is high.

The WHO considers a body mass index (BMI) of 30 or higher to be indicative of obesity. The incidence of this extreme form of being overweight has nearly doubled since 1980 according to the WHO. In 2008, more than 1.4 billion adults, 20 and older, were overweight. Of these over 200 million men and nearly 300 million women were obese. Overweight and obesity are considered leading risks for global deaths. Around 3.4 million adults die each year as a result of being overweight or obese. In addition, 44% of the diabetes burden, 23% of the ischaemic heart disease burden and between 7% and 41% of certain cancer burdens are considered attributable to overweight and obesity.

In view of the increasing prevalence of obesity, of diseases characterised by insulin resistance (such as metabolic syndrome and type 2 diabetes) and diseases characterised by decreased insulin production (such as type 1 diabetes) in the world's population, there is an increasing need for additional treatment opportunities, in particular for treatments suitable to overcome the limitations of current therapeutics. Thus, the technical problem underlying the present invention is the provision of novel means and methods useful in the treatment and/or prevention of obesity, of diseases characterised by insulin resistance (such as metabolic syndrome and type 2 diabetes) or diseases characterised by decreased insulin production (such as type 1 diabetes).

The solution to this problem is achieved by providing the embodiments as characterised in the claims.

Accordingly, the present invention relates to an inhibitor of Hairy and Enhancer of Split 3 (Hes3) for use in a method of preventing or treating (i) a disease characterised by insulin resistance or (ii) obesity.

The following terms are all known in the art and are used in accordance with the definitions provided there. The definitions provided in this specification are solely intended to reflect the understanding of these terms in the art. Where nevertheless a discrepancy arises, the definitions provided herein supersede.

The term "Hes3" is an abbreviation of "Hairy and enhancer of split 3" and both terms are used interchangeably herein. Hes3 (also known as Class B basic helix-loop-helix protein 43 (bHLHb43), GeneID: 390992, GenBank accession no: NP_001019769.1, UniProtKB accession number: Q5TGS1 (as of September 16, 2014 the sequence had last been modified on December 21, 2004)) is a basic helix-loop-helix (bHLH) transcription factor belonging to the Hes/Hey gene family. The sequences can be obtained, for example, in the world wide web, e.g., at ncbi.nlm.nih.gov or at uniprot.org. It is of note that also all the sequences accessible through the respective accession numbers mentioned throughout this application are within the scope of the present invention irrespective of whether the entry of the respective accession number is completely identical to the sequence displayed by the corresponding SEQ ID NO due to potential future updates in the databases. Thus, this is to account for future corrections and modifications in the entries of the databases, which might occur due to the continuing progress of science. Transcription factors of this family regulate developmental processes in progenitor cells from various tissues. Hes3 stands out within this family as an indirect target of a non-canonical branch of the Notch signalling pathway (Poser et al., (2013) Front Physiol 4, 273), whereas other family members such as Hes1 and Hes5 are direct transcriptional targets of the cleaved intracellular domain of the Notch receptor (Guruharsha et al. (2012) Nat Rev Genet 13, 654-666). Specifically, in rodent neural stem cell (NSC) cultures, activation of the Notch receptor by soluble forms of the Delta4 and Jagged1 ligands induces the PI3 kinase - dependent phosphorylation of Akt, mTOR, and STAT3, the latter taking place on serine residue 727 of STAT3 (SEQ ID NOs:1 to 3 corresponding to human (Uniprot: P40763; as of September 16, 2014 the sequence had last been modified on June 7, 2004), mouse (Uniprot: P42227; as of September 16, 2014 the sequence had last been modified on October 1, 1996) and rat (UniProt:P52631; as of September 16, 2014 the sequence had last been modified on October 1, 1996) STAT3).) or on the corresponding serine residue of STAT3 from a different organism. These phosphorylation events are followed by the induction of Hes3 transcription leading to increased cell survival and growth (Androutsellis-Theotokis et al. (2006) Nature 442, 823-826). Another activator of the Akt/mTOR/STAT3-serine pathway, insulin, also induces Hes3 transcription and promotes cell growth (Androutsellis-Theotokis et al. (2008) Cold Spring Harb Symp Quant Biol 73, 403-410). In NSCs the Notch pathway is counteracted by the simultaneous activation of an opposing pathway by Notch cleavage. In this opposing pathway mitogen and stress activated kinase 1 (MSK1) and liver kinase B 1 (LKB1) prevent the phosphorylation of STAT3 at Serine 727. Instead, STAT3 is phosphorylated on tyrosine 705. This opposing pathway can also be accessed via ciliary neurotrophic factor (CNTF) and Leukemia inhibitory factor receptor (LIFR) which induce activation of the Janus protein kinase (JAK) via gp130; in addition, p38 activation further induces JAK activation. NSC cultures from the subventricular zone of adult Hes3 null mice can be established but they are non-responsive to treatments that normally promote Hes3 expression and increase their number such as Delta4 and insulin (Androutsellis-Theotokis et al. (2008) Cold Spring Harb Symp Quant Biol 73, 403-410). The expression of Hes3 has further been shown to induce the expression of Sonic hedgehog (Shh). In addition, it has been described that inhibition of Hes3 expression by RNA interference in cultures of primary human brain cancer stem cells opposes their growth (Park et al. (2013) Sci Rep 3, 1095).

Hes3 has two forms: Hes3a and Hes3b (Hirata et al. (2000) J Biol Chem 275, 19083-19089). Hes3a cannot bind DNA but can still form heterodimers with other basic helix-loop-helix factors (bHLH). Hes3b can both bind DNA and form heterodimers. In some preferred embodiments Hes3 is Hes3a. In other preferred embodiments Hes3 is Hes3b. In addition, a synonymous single nucleotide polymorphisms (SNP) (rs61760837) as well as a non-synonymous SNP (rs61760836) leading to the replacement of a proline by threonine in the orange domain of Hes3 have been described. The rs-numbers are based on the SNP database maintained at NCBI (dbSNP; in the world wide web at ncbi.nlm.nih.gov/SNP/) where each SNP is given a unique SNP identifier consisting of the letters "rs" followed by a specific number. The sequences identified by the respective rs-numbers can also be obtained, for example, in the world wide web at ensemble.org. As used herein the term "Hes3" encompasses all these forms of Hes3. Preferably, Hes3 is a protein which comprises or consists of the amino acid sequence of any one of SEQ ID NOs:4 to 7 or is encoded by a nucleic acid comprising or consisting of any one of SEQ ID NOs:8 to 10. These sequences refer to the amino acid sequence or the nucleic acid sequence of different Hes3 proteins (Table 1). Also encompassed are SNP variants, splice forms, homologous molecules from other species, such as orthologues, such as for example, homologous molecules from pig or primates, or mutated sequences from the same species.

**Table 1: Listing of the different Hes3 amino acid and nucleic acid sequences referred to in the present invention together with the corresponding species.**

| Hes3 variant | species | amino acid sequence (SEQ ID NO) | UniProt accession number and date of last modification to the sequence (as of September 16, 2014) | nucleotide sequence (SEQ ID NO) | NCBI Reference Sequence |
|---|---|---|---|---|---|
| Hes3 | human | 4 | Q5TGS1; December 21, 2004 | 8 | No_001024598.3 |
| Hes3a | mouse | 5 | Q61657; December 12, 2006 | 9 | NM_008237.4 |
| Hes3b | mouse | 6 | Q9JHC5; October 1, 2000 | - | |
| Hes3 | rat | 7 | Q04667; June 1, 1994 | 10 | No_022687.1 |

The term "inhibitor" designates a compound significantly lowering, including completely abolishing (within the limits of detection) the activity of a target molecule, preferably by performing one or more of the following effects: (i)(a) the transcription of the gene encoding the protein to be inhibited is lowered, i.e. the level of mRNA is lowered, (i)(b) the transcription of a gene encoding a protein counteracting the protein to be inhibited is enhanced, (ii)(a) the translation of the mRNA encoding the protein to be inhibited is lowered, (ii)(b) the translation of the mRNA encoding a protein counteracting the protein to be inhibited is enhanced (iii)(a) the stability of the protein to be inhibited is lowered in the presence of the inhibitor (iii)(b) the stability of the protein counteracting the protein to be inhibited is enhanced in the presence of the inhibitor, (iv)(a) the protein to be inhibited performs its biochemical or cellular function with lowered efficiency in the presence of the inhibitor, (iv)(b) the protein counteracting the protein to be inhibited performs its biochemical or cellular function with enhanced efficiency in the presence of the inhibitor.

The term "significantly lowering" or "lowered" refers to a statistically significant decrease and means that the transcription or translation of a gene or the stability or efficiency of a protein is lowered with increasing preference at least 2-fold, at least 5-fold, at least 10-fold, at least 50-fold and at least 100-fold as compared to the transcription or efficiency in the absence of the Hes3 inhibitor. The term "enhanced" means that the transcription of a gene or efficiency of a protein is enhanced with increasing preference at least 2-fold, at least 5-fold, at least 10-fold, at least 50-fold and at least 100-fold as compared to the transcription or efficiency in the absence of the inhibitor.

The term "protein" as used herein describes a group of molecules consisting of more than 30 amino acids and is used interchangeably with the term "polypeptide" herein, whereas the term "peptide" as used herein describes a group of molecules consisting of up to 30 amino acids. The group of peptides and polypeptides are referred to together with the term "(poly)peptide". Also encompassed by the term "(poly)peptide" are fragments of proteins of more than 30 amino acids. The term "fragment of protein" as used herein refers to a portion of a protein comprising at least the amino acid residues necessary to maintain the biological activity of the protein. Preferably, the amino acid chains are linear. (Poly)peptides may further form multimers consisting of at least two identical or different molecules. The corresponding higher order structures of such multimers are correspondingly termed homo- or heterodimers, homo- or heterotrimers etc. Furthermore, peptidomimetics of such (poly)peptides where amino acid(s) and/or peptide bond(s) have been replaced by functional analogues are also encompassed by the invention. Such functional analogues include all known amino acids other than the 20 gene-encoded amino acids, such as selenocysteine. The term "(poly)peptide" also refers to naturally modified (poly)peptides where the modification is effected e.g. by glycosylation, acetylation, phosphorylation and similar modifications which are well known in the art.

It is also well known that (poly)peptides are not always entirely linear. For instance, (poly)peptides may be branched as a result of ubiquitination, and they may be circular, with or without branching, generally as a result of post-translation events, including natural processing events and events brought about by human manipulation which do not occur naturally. Circular, branched and branched circular (poly)peptides may be synthesized by non-translational natural processes and by synthetic methods. The modifications can be a function of how the (poly)peptide is made. For recombinant (poly)peptides, for example, the modifications will be determined by the host cells posttranslational modification capacity and the modification signals in the amino acid sequence. Accordingly, when glycosylation is desired, a (poly)peptide should be expressed in a glycosylating host, generally an eukaryotic cell, for example Cos7, HELA or others. The same type of modification may be present in the same or varying degree at several sites in a given (poly)peptide. Also, a given (poly)peptide may contain more than one type of modification.

As used herein the term "a protein counteracting the protein to be inhibited" refers to a protein which either reduces or abolishes the expression (for example by acting as a transcriptional repressor), the stability (e.g. by targeting the protein to be inhibited for (proteasomal) degradation or by cleaving it into inactive fragments) or the activity (for example by directly binding to the protein to be inhibited thereby blocking its active site) of the protein to be inhibited or which mediates an effect that is opposed to that of the protein to be inhibited. For example, if the protein to be inhibited is a kinase the counteracting protein acting by the latter mechanism may be a phosphatase targeting the same substrate.

As mentioned herein above, an inhibitor can perform one or more of the indicated activities. Accordingly, a compound may fall within only one or within more than one of the following inhibitor classes. Compounds falling in class (i)(a) include compounds interfering with the transcriptional machinery and/or its interaction with the promoter of said gene and/or with expression control elements remote from the promoter such as enhancers. Compounds falling in class (i)(b) include compounds enhancing the activity/efficiency of the transcriptional machinery and/or its interaction with the promoter of said gene and/or with expression control elements remote from the promoter such as enhancers. Compounds of class (ii)(a) comprise antisense constructs and constructs for performing RNA interference (e.g. siRNA) well known in the art (see, e.g. Zamore (2001) Nat Struct Biol. 8(9), 746; Tuschl (2001) Chembiochem. 2(4), 239). Generally, the following holds true: Targeting on the nucleic acid level is often effected by hybridisation to the target sequence. The higher the level of identity of the targeting compound to the complementary sequence of the target nucleic acid, the higher the level of specificity of targeting. Class (ii)(b) encompasses compounds which enhance the translation of an mRNA, for example, by enhancing its stability. Compounds of class (iii)(a) lower the stability of the protein to be inhibited for example by interfering with its correct folding, by targeting it for (proteasomal) degradation and/or by inducing or mediating its cleavage into inactive fragments. Class (iii)(b) encompasses compounds which enhance the stability of a protein counteracting the protein to be inhibited for example by stabilising its correct folding and/or by preventing its (proteasomal) degradation or cleavage into inactive fragments. Compounds of class (iv)(a) interfere with the molecular or cellular function of the protein to be inhibited. As compounds inhibiting the molecular function, active site binding compounds (e.g. a small molecule, an antibody, peptide or aptamer), in particular compounds capable of binding to the active site of protein to be inhibited, are envisaged. This also includes compounds which do not necessarily directly bind to the protein to be inhibited, but still interfere with the cellular activity of the protein, for example by binding to and/or inhibiting the function, or by inhibiting expression of members of the pathway in which the protein to be inhibited is involved. These members may be either upstream or downstream of the protein to be inhibited within the given signalling pathway. Compounds of class (iv)(b) include those which enhance the molecular or cellular function of the protein counteracting the protein to be inhibited. This includes compounds which bind to the protein counteracting the protein to be inhibited, and thereby enhance the activity of the protein counteracting the protein to be inhibited, for example, by maintaining the active conformation of the counteracting protein. In this regard, a biochemical function designates a function of the protein per se, for example, the enzymatic function of a protein. The cellular function of a protein comprises functions of the protein within the cellular context, e.g., a function which may only occur upon interaction with other cellular components, in particular other proteins.

The inhibitor in accordance with the present invention may in certain embodiments be provided as a proteinaceous compound or as a nucleic acid molecule encoding the inhibitor. For example, the nucleic acid molecule encoding the inhibitor may be incorporated into an expression vector comprising regulatory elements, such as for example specific promoters, and thus can be delivered into a cell. Methods for targeted transfection of cells and suitable vectors are known in the art, see for example Sambrook and Russel ("Molecular Cloning, A Laboratory Manual", Cold Spring Harbor Laboratory, N.Y. (2001)). Incorporation of the nucleic acid molecule encoding the inhibitor into an expression vector allows to either selectively or permanently elevate the level of the encoded inhibitor in any cell or a subset of selected cells of a recipient. Thus, a tissue- and/or time-dependent expression of the inhibitor can be achieved, for example, restricted to pancreatic beta cells. In a preferred embodiment, the inhibitor is therefore a beta cell-specific inhibitor. As used herein the term "pancreatic beta cells" refers to cells which are normally located in the pancreas in clusters known as the islets of Langerhans and which produce, store and release insulin.

Preferably, an inhibitor specifically inhibits the activity of its target.

The term "specifically" in this context refers to the capability of an inhibitor to not have an effect or an essential effect on other molecules than the target molecules. In other words, a corresponding inhibitor does not display cross-reactivity or essentially does not display cross-reactivity. In this context, the term "essentially" is meant to refer to an insignificant or negligible effect. The insignificance or negligibility can be based on functional or quantitative parameters. For example, only a minimal amount of cross-reactivity occurs with a different non-target molecule. Preferably, the expression and/or activity of a non-target molecule is inhibited by not more than 20%, such as not more than 15%, preferably by not more than 10%, such as not more than 5%, such as not more than 2%, more preferably by not more than 1% as compared to the expression and/or activity in the absence of the inhibitor. Further it is preferred that the cross-reactivity with a different non-target molecule is associated only with a negligible or insignificant effect, if any. In this regard it is preferred that the biological or cellular function effected or contributed to by the non-target molecule is reduced by less than 20%, such as less than 15%, preferably by less than 10%, such less than 5%, such as less than 2%, more preferably by less than 1% as compared to the biological or cellular function in the absence of the inhibitor. Most preferably, the cross-reactivity with a different non-target molecule is not associated with any effect, i.e. the biological or cellular function effected or contributed to by the non-target molecule is not reduced.

The term "inhibitor of Hes3" encompasses compounds that (1) inhibit components of the signalling pathway upstream of Hes3, (2) that target Hes3 itself, and (3) that inhibit components downstream of Hes3. Also encompassed are (4) compounds that activate components of a pathway preventing Hes3 expression and/or activation.

The above types of compounds inhibiting Hes3 can belong to one or more of the inhibitor classes (i) to (iv) described herein above: (i)(a) the transcription of the gene encoding Hes3 or of a gene encoding a protein effecting or contributing to Hes3 expression and/or activation is lowered or abolished (e.g. the genes for Delta4, jagged 1, Notch, Angiopoietin 2 (Ang2), PI3K, Akt or mTOR, alternatively, the transcription of a gene encoding a factor acting downstream of Hes3 may be lowered or abolished, such factors include, for example, cholecystokinin B (CCKBR) and the small nucleolar RNAs Snord32a, Snord57, Snord53, Snord35a, Snord49b and Snord104; (i)(b) the transcription of a gene encoding a protein preventing Hes3 expression and/or activation or counteracting its activity is enhanced (e.g. the gene encoding gp130, LIFR; JAK, p38, MSK1, LBK1, Angiopoietin 1 (Ang1), PTEN or Hes1)); (ii)(a) the translation of the gene encoding Hes3 or of a gene encoding a protein effecting or contributing to Hes3 expression and/or activation or of a protein acting downstream of Hes3 is lowered or abolished (e.g. the genes for Delta4, jagged 1, Notch, mTOR, Ang2, PI3K, Akt or CCKBR); (ii)(b) the translation of a gene encoding a protein preventing Hes3 expression and/or activation or counteracting its activity is enhanced (e.g. the gene encoding gp130, LIFR; JAK, p38, MSK1, LBK1, Ang1, PTEN or Hes1); (iii)(a) the stability of Hes3 or of a protein effecting or contributing to contributing to Hes3 expression and/or activation or of a protein acting downstream of Hes3 is lowered or abolished (e.g. Delta4, jagged 1, Notch, mTOR, Ang2, PI3K, Akt or CCKBR); (iii)(b) the stability of a protein preventing Hes3 expression and/or activation or counteracting its activity is enhanced (e.g. gp130, LIFR; JAK, p38, MSK1, LBK1 or Hes1), (iv)(a) Hes3 or a protein effecting or contributing to Hes3 expression and/or activation (e.g. Delta4, jagged 1, Notch, Ang2, PI3K, Akt or mTOR) or a protein acting downstream of Hes3 (e.g. CCKBR) performs its biochemical or cellular function with lowered efficiency or has abolished biochemical or cellular function in the presence of the inhibitor; (iv)(b) a protein preventing Hes3 expression and/or activation or counteracting its activity (e.g. gp130, LIFR; JAK, p38, MSK1, LBK1, Ang1, PTEN or Hes1) performs its biochemical or cellular function with enhanced efficiency or has enhanced biochemical or cellular function in the presence of the Hes3 inhibitor.

As described above, compounds of class (ii)(a) comprise antisense constructs and constructs for performing RNA interference (e.g. siRNA) well known in the art (see, e.g. Zamore (2001) Nat Struct Biol. 8(9), 746; Tuschl (2001) Chembiochem. 2(4), 239). In particular antisense constructs and constructs for performing RNA interference which specifically target Hes3, Delta4, jagged 1, Notch, mTOR, PI3K, Akt, Ang2 or CCKBR are envisaged. It is known in the art how such constructs are designed. Exemplary siRNAs for the recited factors can be produced and are therefore commercially available, for example, from Sigma-Aldridge, Qiagen, Santa Cruz or OriGene upon request. In accordance with the present invention the Hes3 inhibitor does not inhibit insulin or insulin receptor. Class (ii)(b) encompasses compounds stabilising the mRNA of gene encoding a protein preventing Hes3 expression and/or activation (e.g. gp130, LIFR; JAK, p38, MSK1, LBK1, PTEN or Ang1) or enhancing its translation in another manner. The translation of mRNA to protein and the stability of the protein itself can be regulated by specific non-coding (nc) RNA. These include micro-RNA, long-non-coding-RNA, circular-RNA and others. Usually, the activity of ncRNA results in a degradation of the respective mRNA and/or protein and thus lowers the amount of the protein. Therefore, an inhibitor of this class may indirectly increase the translation and, thus, the level of a protein by blocking a corresponding ncRNA. Class (iii)(a) includes compounds which lower the stability of Hes3 or of a protein effecting or contributing to Hes3 expression and/or activation or of a protein acting downstream of Hes3, for example by interfering with its correct folding and/or by targeting it for proteasomal degradation. This may include ubiquitin ligases specifically targeting the protein for proteasomal degradation or proteases specifically cleaving the protein into inactive fragments. Compounds falling within class (iii)(b) enhance the stability of a protein preventing Hes3 expression and/or activation. Thus, this class includes, for example, compounds inhibiting ubiquitin ligases or proteases that, in absence of the compound, lead to the degradation or cleavage of the protein preventing Hes3 expression and/or activation. Also encompassed are compounds directly binding to the protein preventing Hes3 expression and/or activation and stabilising its correct folding. Compounds of class (iv)(a) interfere with the molecular or cellular function of Hes3, of a protein effecting or contributing to Hes3 expression and/or activation or of a protein acting downstream of Hes3. Specifically, compounds interfering with activating post-translational modifications such as, for example, phosphorylation are also encompassed. As used herein the term "activating post-translational modification" refers to alternations to a protein which occur after its translation from mRNA into protein has been completed and which contribute to the signal transduction. This encompasses different kinds of modifications, such as for example, folding, cleavage, or addition of chemical groups or small proteins, such as, e.g., phosphorylation and non-degrading types of ubiquitination. Not encompassed are modifications that lead to the inactivation or degradation of the modified protein. Preferably, an activating post-translational modification is a phosphorylation. Accordingly, active site binding compounds (e.g. a small molecule, an antibody, a peptide or an aptamer), for example for mTOR, PI3K or Akt are envisaged. Such inhibitors are known in the art and include for example, the mTOR inhibitors everolimus and temsirolimus. Also envisaged are compounds which block activating post-translational modifications. This includes, for example, compounds which bind to or block the binding sites of the protein thereby blocking these sites for other interaction partners. The latter group of compounds which block binding sites of the protein may be fragments or modified fragments with improved pharmacological properties of the naturally occurring binding partners. For example, small peptides binding specifically to STAT3 phosphorylated at serine 727 of any one of SEQ ID NOs:1) or at the position corresponding thereto and preventing its interaction with downstream signalling components are envisaged. Also dominant-negative versions of proteins are envisaged. Such dominant-negative proteins maintain the ability to bind to interaction partners and thus compete with their naturally occurring active counterpart. At the same time they lack the activity of the naturally occurring variant. For example, a kinase-dead variant of, for example, mTOR, PI3K or Akt may compete with the naturally occurring active protein, but cannot phosphorylate the targets of mTOR thus blocking the signalling pathway. Also envisaged are variants of the pathway components which have been altered so that activating posttranslational modifications are no longer possible. This includes, for example, a variant of STAT3 that cannot be phosphorylated at serine 727 or at a position corresponding thereto in STAT3 from a different organism. Such a variant may be generated by replacing the serine at this site by an alanine. Also encompassed in this class are compounds that interfere with the molecular function of Hes3, e.g. with its ability to translocate to the nucleus, to bind DNA and/or to act as a transcriptional activator or repressor. Class (iv)(b) includes compounds which enhance the molecular or cellular function of a protein preventing Hes3 expression and/or activation. This class may therefore comprise, e.g., Ang1 agonists such as soluble forms of Ang1 which may be recombinantly produced. Further, this class may encompass a variant of STAT3 mimicking constitutive phosphorylation at tyrosine 705 in STAT3 of any one of SEQ ID NOs:1 to 3) or at the corresponding position in STAT3 derived from a different organism. Such a variant can be obtained, for example, by replacing the relevant tyrosine residue with glutamate or aspartate. Preferably, the inhibitor of Hes3 is not an mTOR inhibitor.

The efficiency of a Hes3 inhibitor can be determined by comparison to a negative control and/or a positive control as defined and further detailed herein. As used herein a "positive control" designates compounds which in conditions/experimental setups for Hes3 expression and/or activity show results which are already known or expected to be positive, i.e. a positive control will result in the inhibition of Hes3 expression and/or activity after a predetermined time and may be a known Hes3 inhibitor. An example of a positive control is a Hes3 siRNA. Suitable siRNAs for Hes3 are known in the art and can be obtained commercially, for example, form Santa Cruz Biotechnology or from OriGene Tec. As used herein a "negative control" designates compounds which in conditions/experimental setups for testing Hes3 expression and/or activity show results which are already known or expected to be negative, i.e. a negative control will result in the occurrence of Hes3 expression and/or activity after a predetermined time and may be an inactive compound, e.g. a control siRNA such as, for example, a scrambled siRNA.

As mentioned herein above, a Hes3 inhibitor prevents Hes3 expression and/or activation. In this regard, the term "prevent" includes also reducing Hes3 expression and/or activation which means that the Hes3 expression and/or activation is reduced by the Hes3 inhibitor with increasing preference at least by 20%, at least by 30%, at least by 40%, at least by 50%, at least by 75%, at least by 90% and at least by 95% as compared to the negative control after a predetermined time.

As used herein the term "a predetermined time" specifies a period of time which is sufficient for the induction or inhibition of Hes3 expression and/or activation. It is known in the art how to determine such a time period.

It is of note that whereas the controls are preferably included into the experimental setup, this is by no means necessary. A control may also be, for example, a statistical readout of Hes3 expression and/or activation obtained from control experiments using the above types of compounds previously carried out. A statistical readout is in general obtained by averaging more than one control experiments previously carried out. The average may be obtained by weighting the results from repeating the same control experiment or by weighting the results from different control experiments. Statistical methods for obtaining a statistical readout are well-known in the art. The statistical readout is then compared to results obtained testing a compound for its ability to act as a Hes3 inhibitor.

The determination of the binding of potential inhibitors can be effected in, for example and without limitation, any binding assay, such as a biophysical binding assay, which may be used to identify binding of test molecules prior to performing the functional/activity assay with the inhibitor. Suitable biophysical binding assays are known in the art and comprise fluorescence polarisation (FP) assay, fluorescence resonance energy transfer (FRET) assay and surface plasmon resonance (SPR) assay. Instead of or in addition to assessing the direct interaction of inhibitor and target molecule by binding assays, one may indirectly determine the interaction of the inhibitor with its target molecule by using a suitable read out. For example, in cases where the inhibitor acts by decreasing the expression level of the target protein, the determination of the expression level of the protein can, for example, be carried out on the nucleic acid level or on the amino acid level.

Methods for determining expression on the nucleic acid level include, but are not limited to, northern blotting, PCR, RT-PCR or real RT-PCR.

A northern blot allows the determination of RNA or isolated mRNA in a sample. Northern blotting involves the use of electrophoresis to separate RNA samples by size and detection with a hybridisation probe complementary to part of or the entire target sequence. Initially, typically total RNA extraction from the sample is performed. If desired, mRNA can be separated from said initial RNA sample through the use of oligo (dT) cellulose chromatography to isolate only the RNA with a poly(A) tail. RNA samples are then separated by gel electrophoresis. The separated RNA is then transferred to a nylon membrane through a capillary or vacuum blotting system. After transfer to the membrane, the RNA is immobilised through covalent linkage to the membrane by, e.g., UV light or heat. Then, the RNA is detected using suitably labelled probes and X-ray film and can subsequently be quantified by densitometry. Suitable compositions of gels, buffers and labels are well-known in the art and may vary depending on the specific sample and target to be identified. The above protocol is typical but by no means limiting for a northern blot.

PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl2, 200 µM of each deoxynucleoside triphosphate, 0.5 µl of each primer (10 µM), about 10 to 100ng of template DNA and 1 to 2.5 units of Taq polymerase. The primers for the amplification may be labelled or be unlabelled. DNA amplification can be performed, e.g., with a Applied Biosystems Veriti® Thermal Cycler (Life Technologies Corporation, Carlsbad, CA), C1000™ thermal cycler (Bio-Rad Laboratories, Hercules, CA,), or SureCycler 8800 (Agilent Technologies, Santa Clara, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step, e.g. at 55°C for 1 min as well as a final extension step, e.g. at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, it is well known in the art how to optimise PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerisation of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerising agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746,121, filed Aug. 15, 1991, describes a "homogeneous RT-PCR" in which the same primers and polymerase suffice for both the reverse transcription and the PCR amplification steps, and the reaction conditions are optimised so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT reaction can be performed, for example, in a 20µl reaction mix containing: 0.5 to 1µg total RNA, approximately 10 to 30 pmol of the forward and reverse primer, respectively, 2µl of 10x RT-PCR buffer, 0.2µl AMV reverse transcriptase (25U/µl), 1µl Taq Polymerase (5U/µl), a ribonuclease inhibitor and H2O up to 20µl final volume.

The reaction may be, for example, performed by using the following conditions: The reaction is held at 45°C for 30min to allow for reverse transcription. The reaction temperature is then raised to 94 C° for 5 minutes to stop the reverse transcriptase reaction and to denature the RNA-cDNA duplex. Next, the reaction temperature undergoes 30 to 40 cycles of 94°C for 30 seconds, 60°C for 45 seconds and 72°C for 1-3 minutes. Finally, the reaction temperature is held at 72 C° for 7 minutes for the final extension step, cooled to 15 C°, and held at that temperature until further processing of the amplified sample. Any of the above mentioned reaction conditions may be scaled up according to the needs of the particular case. The resulting products, e.g., are loaded onto an agarose gel and band intensities are compared after staining the nucleic acid molecules with an intercalating dye such as ethidium bromide or SybrGreen. A lower band intensity of the sample treated with the inhibitor as compared to a non-treated sample indicates that the inhibitor successfully inhibits the protein.

Real-time PCR employs a specific probe, in the art also referred to as TaqMan probe, which has a reporter dye covalently attached at the 5' end and a quencher at the 3' end. After the TaqMan probe has been hybridised in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in real time RT-PCR experiments is a DNA intercalating dye such as SybrGreen for monitoring the de novo synthesis of double stranded DNA molecules.

Methods for the determination of the expression of a protein on the amino acid level include, but are not limited to, western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognising the protein of interest. After washing, a second antibody specifically recognising the first antibody and carrying a readout system such as a fluorescent dye is applied. The amount of the protein of interest is determined by comparing the fluorescence intensity of the protein derived from the sample treated with the inhibitor and the protein derived from a non-treated sample. A lower fluorescence intensity of the protein derived from the sample treated with the inhibitor indicates a successful inhibitor of the protein. Also of use in protein quantification is the Agilent Bioanalyzer technique (e.g., Agilent 2100 Bioanalyzer; Agilent Technologies, Santa Clara, CA).

Similarly, the efficiency of the inhibitor, independently of the class it belongs to, with the exception of inhibitors that target components that are downstream of Hes3 and/or its targets, can be quantified by comparing the amount of Shh, CCKBR and/or other targets of Hes3 on the mRNA or protein level in the presence of the inhibitor to that in the absence of the inhibitor. Alternatively, the efficacy of the inhibitor can be assessed or by determining the expression of a reporter gene, such as luciferase or GFP, under the control of the Shh promoter, in presence or absence of the inhibitor, wherein a reduction in the expression of the reporter gene in the presence of or after administration of the inhibitor as compared to the expression in the absence of or prior to said administration is indicative of a successful inhibition of Hes3. Assays for determining the expression of a factor on mRNA- or protein-level are described above.

A reported gene assay for assessing the ability of Hes3 to act as a transcriptional repressor has been described previously (Hirata et al. (2000) J Biol Chem 275, 19083-19089) and can also be used to assess the efficiency of a Hes3 inhibitor.

The efficiency of inhibitors, especially inhibitors of class (iv), can further by assessed by analysing the subcellular localisation of Hes3 in presence versus absence of the inhibitor. The subcellular localisation of Hes3 can be assessed by immunocytochemical analysis or by the use of cell lines transfected with a GFP-fused Hes3 construct. Methods for immunocytochemical analysis are well known in the art and are described, for example, in Immunocytochemistry Methods and Protocols, edited by Lorette C. Javois, 2nd edition, 1999, Human Press, or on the world wide web (www) under dede.invitrogen.com/site/de/de/home/References/Molecular-Probes-The-Handbook.html.

Alternatively, a change in the phenotype of a cell or an organism can be taken as a measure for the efficacy of the inhibitor.

Preferably, the inhibitor of the present invention is comprised in a pharmaceutical composition, preferably further comprising a pharmaceutically acceptable carrier, excipient and/or diluent.

The term "pharmaceutical composition", as used herein, relates to a composition for administration to a patient, preferably a human patient. The pharmaceutical composition comprises at least one, such as at least two, e.g. at least three, in further embodiments at least four such as at last five of the inhibitors mentioned herein. In cases where more than one inhibitor is comprised in the pharmaceutical composition it is understood that none of these inhibitors has any or any essentially inhibitory effect on the other inhibitors also comprised in the composition. The term "essentially" in this context refers to an insignificant or negligible inhibitory effect. Again, it is preferred that the inhibitors provide an additive effect and, optionally, a synergistic effect in their inhibitory activity.

The composition may be in solid, liquid or gaseous form and may be, inter alia, in a form of (a) powder(s), (a) tablet(s), (a) solution(s) or (an) aerosol(s).

As mentioned above, it is preferred that said pharmaceutical composition comprises a pharmaceutically acceptable carrier, excipient and/or diluent. Examples of suitable pharmaceutical carriers, excipients and/or diluents are well known in the art and include phosphate buffered saline solutions, water, emulsions, such as oil/water emulsions, various types of wetting agents, sterile solutions etc. The carriers, excipients and diluents to some extent depend on the chemical nature of the actual inhibitors and can be chosen according to established protocols. Compositions comprising such carriers can be formulated by well known conventional methods. These pharmaceutical compositions can be administered to the subject at a suitable dose. Administration of the suitable compositions may be effected by different ways, e.g., by intravenous, oral, rectal, intraperitoneal, subcutaneous, intramuscular, topical, intradermal, intranasal or intrabronchial administration. It is preferred that the mode of administration is a systemic administration such as, e.g., carried out by injection and/or delivery, e.g., to a site in the bloodstream such as a coronary artery. The compositions may also be administered directly to the target site, e.g., by biolistic delivery to an external or internal target site, preferably the pancreas. Local administration may be advantageous to, e.g., minimise the amount of drug used or decrease the risk of adverse side effects, if any. A combination of systemic and localised administration may be chosen in order to achieve a temporally increased concentration of the inhibitor as defined herein at a specific site in the body. The dosage regimen will be determined by the attending physician and clinical factors. As is well known in the medical arts, dosages for any one patient depend upon many factors, including the patient's size, body surface area, age, the potency and bioavailability of the particular compound to be administered, sex, time and route of administration, general health, and other drugs being administered concurrently. In particular, the potency and mode of action of an inhibitor may dictate not only its dosage, but also its way of administration. Pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 0.01 µg to 10 mg units per kilogram of body weight per minute. The continuous infusion regimen may be completed with a loading dose in the dose range of 1 ng and 10 mg/kg body weight.

In the case of localised delivery of the inhibitors of the present invention to the pancreas, various options exist to achieve said site-specific delivery. For example, the inhibitor(s) may be functionalised in that moieties are attached that specifically target the pancreas and more specifically the pancreatic beta cells. Based on the specific expression of defined antigens by pancreatic beta cells, inhibitors may be attached to antibodies or antibody fragments specifically interacting with the antigen expressed by the cell. Alternatively, the vehicle carrying the inhibitor(s) may be functionalised so that pancreatic beta cells are targeted. Where pancreatic beta cells exhibit particular enzymatic activities or uptake mechanisms, inhibitors can be functionalised in a way that it is converted by the enzymatic activity into a functionally active form or that it is subject to the specific uptake mechanism, respectively.

Efficacy of a treatment can be monitored by periodic assessment. As mentioned herein above, the compositions may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. It is particularly preferred that said pharmaceutical composition comprises further agents known in the art to antagonise heart failure. Since the pharmaceutical preparation relies on the above mentioned inhibitors, it is preferred that those mentioned further agents are only used as a supplement, i.e. at a reduced dose as compared to the recommended dose when used as the only drug, so as to e.g. reduce side effects conferred by the further agents. Conventional excipients include binding agents, fillers, lubricants and wetting agents.

As used herein the term "a disease characterised by insulin resistance" refers to a condition, wherein cells which usually respond to insulin by taking up and storing glucose, such as, for example, liver cells, cells of the skeletal muscle and adipose/fat tissue, become less sensitive and eventually resistant to insulin. The resulting increase in blood glucose may raise levels outside the normal range and causes adverse health effects. Preferably, the disease characterised by insulin resistance is metabolic syndrome or type 2 diabetes.

As used herein the term "type 2 diabetes" refers to a condition characterised by ineffective use of insulin also referred to as insulin resistance. Due to cells such as, for example, liver, skeletal muscle and adipose/fat tissue becoming less sensitive and eventually resistant to insulin, glucose can no longer be absorbed by the cells but remains in the blood. Thus, type 2 diabetes is initially characterised by an increased need for insulin (hyperinsulinaemia). This prolonged increased production of insulin may lead to a degeneration of the beta cells of the pancreas. Thus, at later stages type 2 diabetes is characterised by increased concentrations of glucose in the blood increases (hyperglycaemia). This leads to damage to the body including a build-up of triglycerides which further impairs insulin sensitivity and damage to the body's microvascular system (leading to kidney, eye and nerve damage).

The term "metabolic syndrome" refers to a complex of several interrelated risk factors for diabetes as well as for cardiovascular disease (CVD) and other diseases. Alternative terms are, for example, "syndrome X", "Reaven's syndrome" and in Australia, "CHAOS". The factors of this syndrome include dysglycemia, raised blood pressure, elevated triglyceride levels, low high-density lipoprotein cholesterol levels, and obesity (particularly central adiposity). Patients suffering from metabolic syndrome exhibit resistance to insulin and usually abdominal obesity.

The term "obesity" as used herein refers to a medical condition involving an excessive amount of body fat. A body mass index (BMI) of 30 or higher is considered to be indicative of obesity. Preferably, the term refers to a form of obesity which is associated with at least partial insulin resistance.

The term "prevent" in the context of diseases means that the disease is precluded from developing or delayed in its progress in the presence of the inhibitor for use according to the invention. As will be understood, even a reduction in the risk and/or rate of developing the disease will be beneficial as it may render the disease more manageable from a clinical standpoint and may increase the life expectancy and/or quality of the patient afflicted with the disease. Therefore, the term "prevent" also encompasses the meaning of reducing the risk and/ or rate of developing the disease. In other words, while the term "prevent" thus encompasses precluding the disease, i.e. the disease does not occur, it also relates to a decrease in the risk and/or rate of developing the disease, e.g. by at least (for each value) 30%, 40%, or 50% such as at least (for each value) 55%, 60%, 65%, 70% or 75%, preferred by at least (for each value) 80% or 85%, more preferred at least (for each value) 90% or 95% and most preferred by 100%, i.e. no disease occurs, as mentioned before.

The term "treat" in the context of diseases means that the disease which is already present is eradicated or reduced. This may also encompass eradicating or reducing not the disease as such but only individual symptoms thereof. It will be understood that even reducing severity of the disease or of individual symptoms thereof may increase the life expectancy or overall well-being of the patient afflicted with the diseases. Therefore, the term "treat" also encompasses the meaning of reducing the severity of the disease or of symptoms thereof. In other words, while the term "treat" thus encompasses eradication of the disease or of a symptom thereof, i.e. the patient no longer suffers from the disease or from the symptom, it also relates to a decrease in the severity of the disease or of a symptom thereof, e.g. by at least (for each value) 30%, 40%, or 50% such as at least (for each value) 55%, 60%, 65%, 70% or 75%, preferred by at least (for each value) 80% or 85%, more preferred at least (for each value) 90% or 95% and most preferred by 100%, i.e. the disease or symptom is eradicated and the patient no longer suffers therefrom, as mentioned before.

Preferably, the inhibitor is for use in preventing or treating a disease characterised by insulin resistance or obesity in a mammalian subject. More preferably, the subject is human.

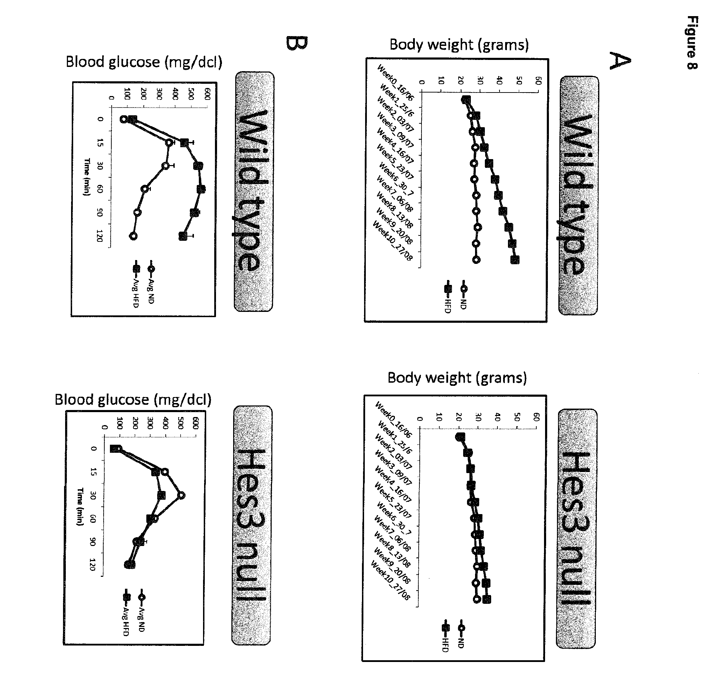

In accordance with the present invention, it was surprisingly found that Hes3 deficient mice gain less weight than wild-type mice when fed a high fat diet (HFD) and that these mice are able to lower their glucose levels efficiently (see Figure 8). This indicates that contrary to wild-type mice, Hes3 null mice do not develop type 2 diabetes. Therefore, inhibition of Hes3 is a suitable approach in preventing and/or treating diseases associated with insulin resistance. Examples of such diseases are metabolic syndrome and type 2 diabetes. Further, as mentioned previously, Hes3 deficiency surprisingly also reduced the weight gain induced by the HFD significantly thus making inhibition of Hes3 a suitable option also for prevention and/or treatment of obesity. The present invention thus provides advantageous treatment opportunities for diseases for which additional treatments are urgently needed due to their increasing worldwide prevalence.

The invention further relates to a method for preventing or treating (i) a disease characterised by insulin resistance or (ii) obesity, comprising administering a therapeutically effective amount of an inhibitor of Hes3 to a subject in need thereof.

The definitions and preferred embodiments provided for the inhibitor for use according to the invention apply *mutatis mutandis* also to this method of the invention.

The term "comprising" in the context of the methods of the invention denotes that the methods may include further steps. Alternatively, the methods consist of the specified steps, i.e. no other steps are performed in addition to the steps described herein.

With regard to the method of preventing or treating a disease characterised by insulin resistance or obesity the term "therapeutically effective amount" refers to a quantity of the Hes3 inhibitor that is sufficient to prevent or treat a disease characterised by insulin resistance or obesity. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g per day. However, a more preferred dosage might be in the range of 0.01 mg to 100 mg, even more preferably 0.01 mg to 50 mg and most preferably 0.01 mg to 10 mg per day.

In the context of method of preventing or treating a disease characterised by insulin resistance or obesity the term "subject in need thereof" refers to an animal, preferably a vertebrate, more preferably a mammal, and most preferably to a human subject which suffers from a disease characterised by insulin resistance or obesity.

In a preferred embodiment of the inhibitor for use according to the invention or of the above method of the invention, the inhibitor of Hes3 is to be used (a) as the sole pharmaceutically active ingredient; or (b) in combination with a further therapeutic agent, preferably a further therapeutic agent for the same medical indication.

In accordance with this embodiment the term "as the sole pharmaceutically active ingredient" indicates that the Hes3 inhibitor is the only substance preventing or treating the disease characterised by insulin resistance or obesity or suspected of doing so. This does not exclude more than one Hes3 inhibitor being included. In other words, two or more Hes3 inhibitors belonging to the same or different classes of inhibitors as described herein may be present. Further, the Hes3 inhibitor(s) may nevertheless be provided in a pharmaceutical composition, comprising additional ingredients, which however are on their own ineffective in preventing or treating the disease characterised by insulin resistance or obesity. This includes, for example, a pharmaceutically acceptable carrier, an excipient and/or a diluent as defined herein above. Further, the concomitant use of a therapeutic agent for a different medical indication is not excluded.

The term "in combination with a further therapeutic agent" refers to the Hes3 inhibitor having admixed thereto another pharmaceutically effective ingredient suitable in preventing or treating the disease characterised by insulin resistance or obesity. Also encompassed is a combination of a Hes3 inhibitor with such an additional pharmaceutically effective ingredient wherein the two or more active ingredients are not admixed but are provided separately. In that case the Hes3 inhibitor and the additional pharmaceutically effective agent(s) are to be administered simultaneously or within a short period of time of each other. Administration may occur by the same or by different routes in this case. Preferred therapeutic agents to be combined with the Hes3 inhibitor are therapeutic agents for the same medical indication, i.e. therapeutics, in particular established therapeutics, for the treatment a disease characterised by insulin resistance or obesity. In particular, a combination of an Hes3 inhibitor with an established therapeutic for the treatment of type 2 diabetes, such as for example, a sulfonylurea, a biguanide, a sodium-glucose co-transporter (SGLT2) inhibitor, a thiazolidinedione, an alpha glucosidase inhibitor, an amylin analogue, an incretin mimetic, a glucagon-like peptide-1 (GLP-1) agonist, a DPP-IV inhibitor, a combination medicament or with insulin is envisaged. More specifically, a Hes3 inhibitor may be combined with one or more of the following drugs: metformin, pioglitazone, exenatide, sitagliptin, colesevelam, a combination of sitagliptin and metformin, glipizide, glimepiride, insulin glargine, glibenclamide (also known as glyburide), canagliflozin, rosiglitazone, saxagliptin, insulin detemir, a combination of pioglitazone and metformin, linagliptin, an extended release formulation comprising saxagliptin and metformin HCl, bromocriptine, liraglutide, repaglinide, insulin lispro, nateglinide, acarbose, pramlintide, dapagliflozin, a combination of rosiglitazone maleate and glimepiride, insulin glulisine, miglitol, chlorpropamide, alogliptin, tolbutamide, tolazamide, mifepristone, a combination of rosiglitazone and metformin, a combination of repaglidine and metformin, a combination of linagliptin and metformin, a combination of pioglitazone and glimepiride, a combination of alogliptin and pioglitazone and a combination of alogliptin and metformin.

In addition, the invention also relates to a pharmaceutical composition comprising Hes3.

The definition of the term "Hes3" as provided with regard to the inhibitor for use according to the invention applies *mutatis mutandis* also to the pharmaceutical composition of the invention. In particular, in the context of the pharmaceutical composition of the invention the term encompasses a Hes3 protein as well as a nucleic acid molecule encoding Hes3.

As defined herein above, with regard to the inhibitor for use according to the invention the term "pharmaceutical composition" relates to a composition for administration to a patient, preferably a human patient. Preferably, the pharmaceutical composition of the invention, in addition to Hes3, comprises a pharmaceutically acceptable carrier, excipient and/or diluent. Examples of suitable pharmaceutical carriers, excipients and/or diluents have been provided herein above. In addition, suitable forms as well as modes of administration and dosage regimes have also been described herein above. With regard to Hes3 for use as a medicament it is preferred that this medicament is used in treating or preventing a disease that is known or thought to be caused or exacerbated by a lack of Hes3 expression and/or activity or that is thought to be treatable or preventable by increasing the expression or activity of Hes3. In particular, diseases characterised by decreased insulin production, such as for example, type 1 diabetes, are envisaged. Also envisaged is the treatment of gestational diabetes. It is preferred that the disease to be treated is type 1 diabetes.

The invention also relates to Hes3 and/or an activator thereof for use in a method of preventing or treating a disease characterised by decreased insulin production wherein said activator of Hes3 is (a) an antibody, an antibody mimetic, a dominant negative protein, an siRNA, an shRNA, an miRNA, a ribozyme, an aptamer, an antisense nucleic acid molecule or a small molecule inhibiting Janus protein tyrosine kinase (JAK); (b) an antibody, an antibody mimetic, a dominant negative protein, an siRNA, an shRNA, an miRNA, a ribozyme, an aptamer, an antisense nucleic acid molecule or a small molecule inhibiting p38; (c) a variant of STAT3 that cannot be phosphorylated on tyrosine 705; (d) a variant of STAT3 that mimics constitutive phosphorylation on serine 727; (e) a soluble variant of Delta4 or jagged 1; (f) an agonistic antibody specifically binding to Notch; or (g) an inhibitor of leptin signalling.

The definitions and preferred embodiments provided herein above with regard to the pharmaceutical composition of the invention apply *mutatis mutandis* also to Hes3 and/or an activator thereof for use according to the invention.

As used herein the term "activator of Hes3" refers to means and methods inducing or enhancing the expression, activation or activity of Hes3. In other words, an activator of Hes3 may, for example, increase the transcription, the translation or the stability of Hes3 and/or it may induce or enhance its translocation to the nucleus, its binding to interaction partners, its binding to DNA or its ability to act as a transcriptional activator or repressor.

As used herein, the term "disease characterised by decreased insulin production" refers to conditions, wherein the ability of the pancreatic beta cells to produce insulin is reduced or abolished. This may be due to the cells not working properly, i.e. being present but functionally impaired, or to the cells being destroyed. Preferably, the term refers to diseases where the beta cells of the pancreas are destroyed, for example by an aberrant reaction of the immune system. Most preferably, the term refers to type 1 diabetes. Although in later stages of type 2 diabetes the function of the pancreatic beta cells may be impaired so that insulin production is reduced, type 2 diabetes is not encompassed by the term "disease characterised by decreased insulin production" as used herein.

The term "antibody" as used herein comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments, Fd, F(ab')₂, Fv or scFv fragments, single domain V_{H} or V-like domains, such as VhH or V-NAR-domains, as well as multimeric formats such as minibodies, diabodies, tribodies or triplebodies, tetrabodies or chemically conjugated Fab'-multimers (see, for example, Altshuler EP, Serebryanaya DV, Katrukha AG. 2010, Biochemistry (Mosc)., vol. 75(13), 1584 Holliger P, Hudson PJ. 2005, Nat Biotechnol., vol. 23(9), 1126). The term "antibody" also includes embodiments such as chimeric (human constant domain, non-human variable domain), single chain and humanised (human antibody with the exception of non-human CDRs) antibodies.

Various techniques for the production of antibodies are well known in the art and described, e.g. in Altshuler et al., 2010 (Altshuler EP, Serebryanaya DV, Katrukha AG. 2010, Biochemistry (Mosc)., vol. 75(13), 1584). Thus, polyclonal antibodies can be obtained from the blood of an animal following immunisation with an antigen in mixture with additives and adjuvants and monoclonal antibodies can be produced by any technique which provides antibodies produced by continuous cell line cultures. Examples for such techniques are described, e.g. in Harlow E and Lane D, Cold Spring Harbor Laboratory Press, 1988; Harlow E and Lane D, Using Antibodies: A Laboratory Manual, Cold Spring Harbor Laboratory Press, 1999 and include the hybridoma technique originally described by Köhler and Milstein, 1975, the trioma technique, the human B-cell hybridoma technique (see e.g. Kozbor D, 1983, Immunology Today, vol.4, 7; Li J, et al. 2006, PNAS, vol. 103(10), 3557) and the EBV-hybridoma technique to produce human monoclonal antibodies (Cole et al., 1985, Alan R. Liss, Inc, 77-96). Furthermore, recombinant antibodies may be obtained from monoclonal antibodies or can be prepared *de novo* using various display methods such as phage, ribosomal, mRNA, or cell display. A suitable system for the expression of the recombinant (humanised) antibodies may be selected from, for example, bacteria, yeast, insects, mammalian cell lines or transgenic animals or plants (see, e.g., US patent 6,080,560; Holliger P, Hudson PJ. 2005, Nat Biotechnol., vol. 23(9), 11265). Further, techniques described for the production of single chain antibodies (see, inter alia, US Patent 4,946,778) can be adapted to produce single chain antibodies specific for an epitope of a component of the pathway. Surface plasmon resonance as employed in the BIAcore system can be used to increase the efficiency of phage antibodies.

As used herein the term "antibody mimetics" refers to compounds which, like antibodies, can specifically bind antigens, but which are not structurally related to antibodies. Antibody mimetics are usually artificial peptides or proteins with a molar mass of about 3 to 20 kDa. For example, an antibody mimetic may be selected from the group consisting of affibodies (which are based on the Z-domain of staphylococcal protein A) adnectins (based on the tenth domain of human fibronectin), anticalins (derived from lipocalins), DARPins (derived from ankyrin repeat proteins), avimers (based e.g. on multimerised Low Density Lipoprotein Receptor (LDLR)-A), nanofitins (derived from the DNA binding protein Sac7d of *Sulfolobus acidocaldarius*), affilins (structurally derived from gamma-B crystalline or ubiquitin), Kunitz domain peptides (derived from the Kunitz domains of various protease inhibitors) and Fynomers^{®} which are derived from the human Fyn SH3 domain (see e.g. Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12). These polypeptides are well known in the art and will be described in further detail herein below.

As used herein, the term "DARPin" refers to a designed ankyrin repeat domain (166 residues), which provides a rigid interface arising from typically three repeated β-turns. DARPins usually carry three repeats corresponding to an artificial consensus sequence, whereby six positions per repeat are randomised. Consequently, DARPins lack structural flexibility (Gebauer and Skerra, 2009).

A "nanofitin" (also known as affitin) is an antibody mimetic protein that is derived from the DNA binding protein Sac7d of *Sulfolobus acidocaldarius.* Nanofitins usually have a molecular weight of around 7kDa and are designed to specifically bind a target molecule by randomising the amino acids on the binding surface (Mouratou B, Béhar G, Paillard-Laurance L, Colinet S, Pecorari F., (2012) Methods Mol Biol.; 805:315-31).

The term "anticalin" as used herein refers to an engineered protein derived from a lipocalin (Beste G, Schmidt FS, Stibora T, Skerra A. (1999) Proc Natl Acad Sci USA. 96(5):1898-903; Gebauer and Skerra (2009) Curr Opinion in Chemical Biology 13:245-255). Anticalins possess an eight-stranded β-barrel which forms a highly conserved core unit among the lipocalins and naturally forms binding sites for ligands by means of four structurally variable loops at the open end. Anticalins, although not homologous to the IgG superfamily, show features that so far have been considered typical for the binding sites of antibodies: (i) high structural plasticity as a consequence of sequence variation and (ii) elevated conformational flexibility, allowing induced fit to targets with differing shape.

An "adnectin" (also referred to as "monobody") as used herein, is based on the 10th extracellular domain of human fibronectin III (10Fn3), which adopts an Ig-like β-sandwich fold of 94 residues with 2 to 3 exposed loops, but lacks the central disulphide bridge (Gebauer and Skerra (2009) Curr Opinion in Chemical Biology 13:245-255). Adnectins with the desired target specificity can be genetically engineered by introducing modifications in specific loops of the protein.

The term "affibody" as used herein refers to a family of antibody mimetics which is derived from the Z-domain of staphylococcal protein A. Structurally, affibody molecules are based on a three-helix bundle domain which can also be incorporated into fusion proteins. In itself, an affibody has a molecular mass of around 6kDa and is stable at high temperatures and under acidic or alkaline conditions. Target specificity is obtained by randomisation of 13 amino acids located in two alpha-helices involved in the binding activity of the parent protein domain (Feldwisch J, Tolmachev V.; (2012) Methods Mol Biol. 899:103-26).

The term "affilin" as used herein refers to antibody mimetics that are developed by using either gamma-B crystalline or ubiquitin as a scaffold and modifying amino-acids on the surface of these proteins by random mutagenesis. Selection of affilins with the desired target specificity is effected, for example, by phage display or ribosome display techniques. Depending on the scaffold, affilins have a molecular weight of approximately 10 or 20kDa. As used herein the term affilin also refers to di- or multimerised forms of affilins (Weidle UH, et al., (2013), Cancer Genomics Proteomics;10(4):155-68).

The term "avimer" as used herein refers to a class of antibody mimetics which consist of two or more peptide sequences of 30 to 35 amino acids each which are derived from A-domains of various membrane receptors and which are connected by linker peptides. Binding of target molecules occurs via the A-domain and domains with the desired binding specificity can, for example, be selected by phage display techniques. Binding specificity of the different A-domains contained in an avimer may, but does not have to, be identical (Weidle UH, et al., (2013), Cancer Genomics Proteomics;10(4):155-68).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDA and domains with the required target specificity can be selected by display techniques such as phage display (Weidle et al., (2013), Cancer Genomics Proteomics;10(4):155-68).

As used herein the term "Fynomer^{®}" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

The term "dominant negative protein" as used herein refers to versions of proteins which maintain the ability to interact with the interaction partners of the corresponding naturally occurring protein but which lack at least one aspect of the activity of the corresponding naturally occurring protein. Thus, a dominant negative protein is a protein competing with the corresponding naturally occurring protein for interaction partners but lacking its activity. Examples of dominant negative proteins are inactive variants of Hes3 or of proteins effecting or contributing to the expression and/or activation of Hes3. This includes soluble variants of the receptors such as, for example, Notch lacking the transmembrane domain. These receptor-like proteins can still bind to the natural ligand thus preventing its binding to the transmembrane receptors. At the same time the soluble receptor cannot pass on the signal into the cell and thus cannot induce expression and/or activation of Hes3. It is understood that also membrane-anchored receptors can act as a dominant negative protein as long as they can bind to the natural ligand and are unable to intracellularly transmit the signal. This also encompasses soluble forms of receptors generated by cleavage of the extracellular domain, naturally occurring alternative forms of a receptor or other receptors binding to the same endogenous ligand, thus preventing its binding to the receptor to be inhibited, but being unable to transmit the signal which the ligand would induce upon binding to the receptor to be inhibited. Such receptors are also referred to as decoy-receptors. Examples for other dominant negative proteins are kinase-dead variants of mTOR as well as a variant of STAT3 that cannot be phosphorylated at serine 727 of STAT3 of any one of SEQ ID NOs:1 to 3 or at the position corresponding thereto in STAT3 from a different organism.

As used herein, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery in vivo through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics). The activity and specificity of siRNAs can be altered by various modifications such as by inclusion of a blocking group at the 3' and 5' ends, wherein the term "blocking group refers to substituents of that can be attached to oligonucleotides or nucleomonomers, either as protecting groups or coupling groups for synthesis (cf. WO 98/13526, EP 2221377 B1), by inclusion of agents that enhance the affinity to the target sequence such as intercalating agents (e.g., acridine, chlorambucil, phenazinium, benzophenanthirdine), attaching a conjugating or complexing agent or encapsulating it to facilitate cellular uptake, or attaching targeting moieties for targeted delivery.

A short hairpin RNA (shRNA) is a sequence of RNA that makes a tight hairpin turn that can be used to silence gene expression via RNA interference. Experimentally, shRNA uses a vector introduced into cells and utilises the U6 promoter to ensure that the shRNA is expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it.

si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterised small self-cleaving RNAs are the hammerhead, hairpin, hepatitis delta virus, and in vitro-selected lead-dependent ribozymes, whereas the group I intron is an example for larger ribozymes. The principle of catalytic self-cleavage is well established in the art. The hammerhead ribozymes are characterised best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity in vitro and in some cases also in vivo. The best results are usually obtained with short ribozymes and target sequences.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of *in-vitro* selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys- loop in the wild protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, fusion to albumin or other half life extending proteins etc. are available such that the half-life of aptamers can be increased for several days or even weeks.

Also useful is the combination of an aptamer recognising a small compound with a hammerhead ribozyme. The conformational change induced in the aptamer upon binding the target molecule is supposed to regulate the catalytic function of the ribozyme.

The term "antisense nucleic acid molecule" as used herein refers to a nucleic acid which is complementary to a target nucleic acid. An antisense molecule is capable of interacting with the target nucleic acid, more specifically it is capable of hybridising with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

A small molecule - in all embodiments of the present invention - may be an organic or an inorganic small molecule. Organic small molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively the small molecule may be an inorganic small molecule. Inorganic small molecules are derived from mineral sources and include all compounds without carbon atoms (except carbon dioxide, carbon monoxide and carbonates).Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity can be identified and verified in *in-vivo* assays such as *in-vivo* high-throughput screening (HTS) assays. Preferably, the small molecule is an organic small molecule.

As used herein, the term "JAK" is an abbreviation for "Janus protein tyrosine kinase" and both terms are used interchangeably herein. JAK is a non-receptor tyrosine kinase which has been implicated, for example, in a pathway counteracting the expression and activation of Hes3. Therefore, a compound inhibiting the expression and/or activity of JAK is an indirect activator of Hes3. Known JAK inhibitors include, for example, tofacitinib, ruxolitinib, baricitinib, CYT387, GLPG0634, GSK2586184, lestaurtinib, pacritinib and TG101348.

The term "p38" as used herein refers to p38 mitogen-activated protein kinases. Like JAK, p38 has been described to be a component of a pathway counteracting the expression and activation of Hes3. Therefore, compounds inhibiting p38 can be used as activators of Hes3. Known inhibitors of p38 include, for example, pamapimod, PH-797804, BIRB 796, VX-702, SB 239063, SB202190, SCIO 469, and BMS 582949. These inhibitors are known in the art.

As used herein the term "a variant of STAT3 that cannot be phosphorylated on tyrosine 705" refers to a form of STAT3 wherein the amino acid sequence has been altered such that the protein is no longer phosphorylatable at position 705 (see SEQ ID NOs:1 to 3). Also encompassed are variants of STAT3 from other organisms wherein the amino acid sequence has been altered such that the protein can no longer be phosphorylated at the position corresponding to position 705 of any one of SEQ ID NOs:1 to 3. It is known in the art how such a corresponding position can be determined. For example, an alignment may be used to determine the corresponding position in STAT3 from a different organism. Alterations to the amino acid sequence that are suitable to prevent phosphorylation at a given position are also known in the art. For example, the tyrosine at position 705 of STAT3 of any one of SEQ ID NOs:1 to 3 or at a position corresponding thereto in STAT3 from another organism may be replaced by a phenylalanine. Methods for replacing one amino acid by another are well known in the art and include, for example, generating a nucleic acid molecule encoding the protein wherein the nucleotides encoding the relevant amino acid are exchanged, e.g. by site directed mutagenesis. Preferably, the variant of STAT3 that cannot be phosphorylated on tyrosine 705 or a position corresponding thereto is provided in a sufficient amount enabling it to effectively compete with endogenous STAT3 which can be phosphorylated at that position so as to prevent interaction of phosphorylatable STAT3 with its interaction partners. It is also preferred that the variant of STAT3 is provided in the form of a nucleic acid molecule encoding the variant of STAT3.

The term "a variant of STAT3 that mimics constitutive phosphorylation on serine 727" as used herein denotes a form of STAT3 wherein the amino acid sequence has been altered such that the activities of this protein correspond to those of a corresponding STAT3 which is constitutively phosphorylated at position 727 (see any one of SEQ ID NOs:1 to 3). Also encompassed are variants of STAT3 from other organisms wherein the amino acid sequence has been altered such that the protein mimics constitutive phosphorylation at the position corresponding to position 727 of any one of SEQ ID NOs:1 to 3. It is known in the art that such a corresponding position can be determined, for example, by an alignment. Alterations to the amino acid sequence that are suitable to prevent phosphorylation at a given position are also known in the art. For example, the serine at position 727 of STAT3 of any one of SEQ ID NOs:1 to 3 or at a position corresponding thereto in STAT3 from another organism may be replaced by a glutamate or an aspartate residue. Methods for replacing one amino acid by another are well known in the art and have been referred to herein above. Preferably, the variant of STAT3 that mimics constitutive phosphorylation on serine 727 or a position corresponding thereto is provided in the form of a nucleic acid molecule encoding the variant of STAT3.

As used herein the term "a soluble variant of Delta4 or jagged 1" refers to a non-membrane bound form of these ligands. Such a soluble variant may comprise the entire extracellular domain of the respective ligand or only the portion thereof which is required for binding to the receptor Notch. The variant may consist of the extracellular domain or of the receptor-binding portion thereof or it may comprise additional subsequences. Such additional subsequences may include, for example, a tag used for purification or detection of the variant. Preferably, the soluble variant of Delta4 or jagged 1 is a recombinant purified protein.

The term "an agonistic antibody specifically binding to Notch" denotes an antibody which binds to the receptor Notch without binding to any other factor or which binds to other factors only with substantially lower affinity and which induces activation of Notch. For example, an agonistic antibody for Notch is known in the art (Li et al. (2008), JBC 283 (12): 8046-54).

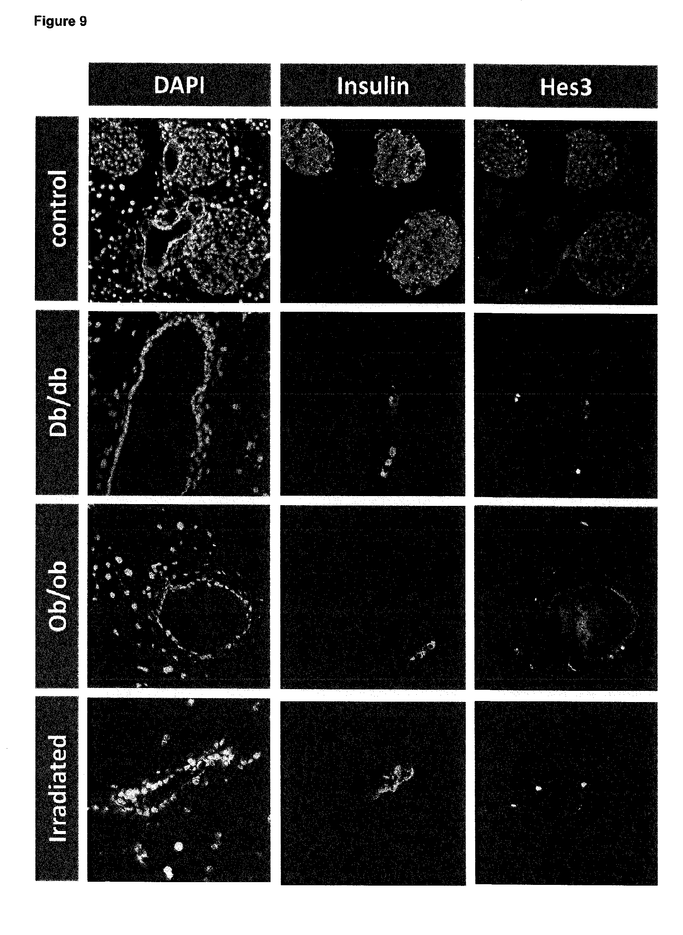

The term "an inhibitor of leptin signalling" refers to a compound interfering with the signal transduction pathway induced by the hormone leptin specifically in the pancreas. Such an inhibitor may for example interfere with the expression and/or activity of one of the pathway components such as, for example, leptin itself, a leptin receptor such as Ob-Rb or of a downstream component such as components of the Jak-Stat and MAPK signal transduction pathways in the pancreas. Preferably, inhibition of leptin signalling specifically in the pancreas is achieved by local administration of the inhibitor to the pancreas and/or by localised delivery of the inhibitors of the present invention to the pancreas. Various options exist to achieve said site-specific delivery. Suitable methods of local administration and methods for targeting a compound to a specific tissue or cell type, in this case the pancreas, are known in the art and have been described herein above. It has been observed in accordance with the present invention that Hes3 is induced in mice where leptin signalling is genetically disrupted (ob/ob and db/db mouse strains), indicating that pancreas-specific inhibitors of leptin signalling are activators of Hes3 expression (see Figure 9).

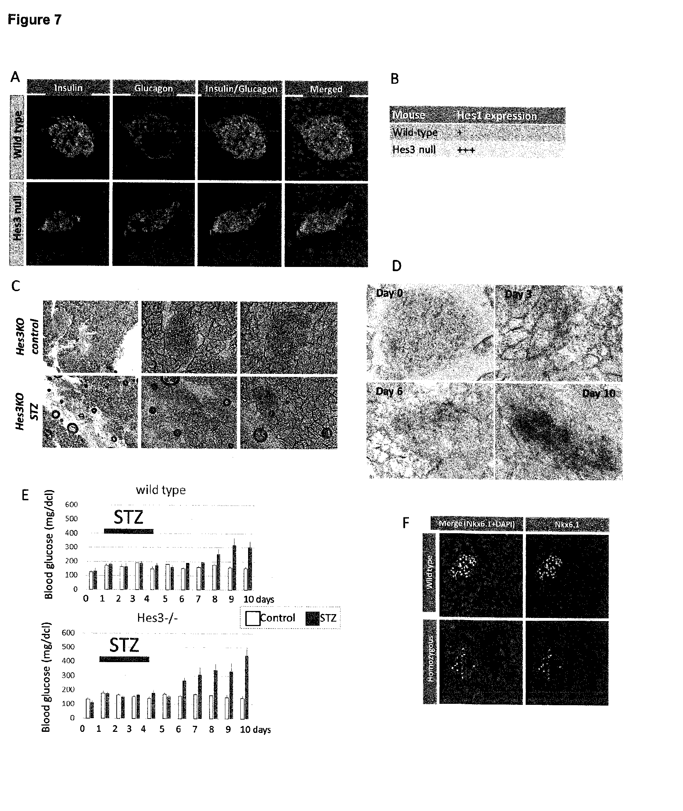

In accordance with the present invention it was surprisingly found that treatment with streptozotocin (STZ) which is used to experimentally model type 1 diabetes by specifically damaging the beta cells of the pancreas induces activation of the Hes3a promoter (the mouse strain employed in these experiments only allows the assessment of Hes3a promoter activity). This suggests a role of Hes3 in this damage paradigm (see Example 8 and Figure 7). In line with this notion, mice lacking Hes3 (Hes3 null mice) showed a greater deficit in beta cells and a faster and more pronounced increase blood glucose level upon treatment with STZ. This indicates that Hes3 is required in the protection and/or regeneration from STZ induced damage. Considering that STZ treatment is an established model for type 1 diabetes, this supports a protective role for Hes3 with regard to type 1 diabetes. Without wishing to be bound by scientific theory it is hypothesised that treatment with Hes3 and/or an activator thereof prevents damage to the beta cells of the pancreas and thus prevents or at least delays the onset of type 1 diabetes. Further, it was shown in accordance with this invention that in mice with disrupted leptin signalling (ob/ob and db/db mouse strains) or in mice subjected to total body irradiation, Hes3 expression can be induced outside the pancreatic islets such as, for example in the pancreatic duct and that the cells expressing Hes3 also express insulin. These data indicate that, even after the onset of type 1 diabetes, Hes3 and/or an activator thereof can be used to initiate neogenesis, i.e. to generate pancreatic islets from non-pancreatic islets cells and to thus rescue or at least partially increase insulin production. Without wishing to be bound by theory, it is further hypothesised that leptin signalling fulfils different functions in different tissues. Therefore, genetic deficiency of a component of leptin signalling throughout the body induces obesity and, potentially indirectly, type 2 diabetes. It is hypothesised that this function is mediated mainly via leptin signalling in the brain. The data provided herein indicate that leptin signalling has the additional role of regulating Hes3 expression and insulin production in the pancreas. Inhibition of leptin signalling in the pancreas induces Hes3 expression and insulin production. Accordingly, compounds inhibiting leptin signalling specifically in the pancreas (but not in other tissues and especially not in the brain) can be used in methods for treating or preventing diseases characterised by decreased insulin production, such as, for example, type 1 diabetes. Thus, treatment opportunities for preventing and/or treating diseases characterised by decreased insulin production, in particular type 1 diabetes, are provided.

Further, the invention relates to a method for preventing or treating a disease characterised by decreased insulin production comprising administering a therapeutically effective amount of Hes3 and/or an activator thereof to a subject in need thereof, wherein said activator of Hes3 is (a) an antibody, an antibody mimetic, a dominant negative protein, an siRNA, an shRNA, an miRNA, a ribozyme, an aptamer, an antisense nucleic acid molecule or a small molecule inhibiting Janus protein tyrosine kinase (JAK); (b) antibody, an antibody mimetic, a dominant negative protein, an siRNA, an shRNA, an miRNA, a ribozyme, an aptamer, an antisense nucleic acid molecule or a small molecule inhibiting p38; (c) a variant of STAT3 that cannot be phosphorylated on tyrosine 705; (d) a variant of STAT3 that mimics constitutive phosphorylation on serine 727; (e) a soluble variant of Delta4 or jagged 1; (f) an agonistic antibody specifically binding to Notch; or (g) an inhibitor of leptin signalling.

The definitions and preferred embodiments provided with regard to the pharmaceutical composition of the invention and with regard to Hes3 and/or an activator thereof for use according to the invention herein above, apply *mutatis mutandis* also to this method of the invention.

With regard to the method of preventing or treating a disease characterised by decreased insulin production the term "therapeutically effective amount" refers to a quantity of Hes3 and/or the activator thereof that is sufficient to prevent or treat a disease characterised by decreased insulin production. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

In the context of method of preventing or treating a disease characterised by decreased insulin production the term "subject in need thereof' refers to an animal, preferably a vertebrate, more preferably a mammal, and most preferably to a human subject which suffers from a disease characterised by decreased insulin production, such as, for example, type 1 diabetes.

In a preferred embodiment of the pharmaceutical composition of the invention, of Hes3 and/or the activator thereof for use according to the invention or of the method for preventing or treating a disease characterised by decreased insulin production Hes3 and/or the activator thereof is/are to be used (a) as the sole pharmaceutically active ingredient; or (b) in combination with a further therapeutic agent.

In accordance with this embodiment the term "as the sole pharmaceutically active ingredient" indicates that Hes3 or the activator thereof is the only substance preventing or treating the disease characterised by decreased insulin production or suspected of doing so. This does not exclude more than one Hes3 activator being included in the presence or absence of Hes3. In other words, Hes3 and/or one or more Hes3 activators as defined herein above may be present. Further, Hes3 and/or the Hes3 activator(s) may nevertheless be provided in a pharmaceutical composition, comprising additional ingredients, which are on their own ineffective in preventing or treating the disease characterised by decreased insulin production. This includes, for example, a pharmaceutically acceptable carrier, excipient and/or diluent as defined herein above.

The term "in combination with a further therapeutic agent" refers to Hes3 or the activator thereof having admixed thereto another pharmaceutically effective ingredient suitable in preventing or treating the disease characterised by decreased insulin production. Also encompassed is a combination of Hes3 and/or an activator thereof with such an additional pharmaceutically effective ingredient wherein the two active ingredients are not admixed but are provided separately. In that case Hes3 and/or an activator thereof and the additional pharmaceutically effective agent are to be administered simultaneously or within a short period of time of each other. Administration may occur by the same or by different routes in this case. Preferred therapeutic agents to be combined with the Hes3 and/or an activator thereof are therapeutic agents for the same medical indication, i.e. therapeutics, in particular established therapeutics, for the treatment a disease characterised by decreased insulin production. In particular, a combination of Hes3 and/or an activator thereof with an established therapeutic for the treatment of type 1 diabetes, such as for example insulin, is envisaged.

In addition, the invention also relates to the use of Hes3 as a target in a screen for identifying a compound suitable as a lead compound and/or as a medicament for the treatment and/or prevention of (a) diseases characterised by insulin resistance or of obesity; or (b) diseases characterised by decreased insulin production.

In accordance with this embodiment the term "compound" denotes any substance to be tested for its suitability either in treating or preventing a disease characterised by insulin resistance or obesity or in treating or preventing a disease characterised by reduced insulin production. The compound or may be a compound that is designed or expected to target Hes3 or an upstream or downstream component thereof. However, any substance may be tested, i.e. also substances which are not specifically designed to target a particular component.

The term "screen" as used herein refers to an assay determining whether a compound has a specific property. Usually, a large number of compounds are analysed for the presence or absence of the specific property or properties. Encompassed are in particular assays assessing the binding of a compound to a target (binding assays) and assays determining the capability of a compound to inhibit or enhance the activity of the target respectively (functional assays). In particular, it is envisaged that the compound may be analysed for its ability to bind to the target prior to being subjected to the functional analysis. Binding assays may be performed, for example, by a biophysical binding assay such as fluorescence polarisation (FP) assay, fluorescence resonance energy transfer (FRET) assay or surface plasmon resonance (SPR) assay as mentioned herein above. Further, DNA-encoded chemical libraries (DEL) may be employed in identifying high affinity binders for a given target. Such libraries and their use are known in the art. Briefly, such a DEL comprises a large number of small molecules, each of which is liked to a short identifier DNA sequence. The DEL is subjected to affinity selection on an immobilized target, such as a protein, which in the present case may be Hes3, unbound molecules are removed by a washing step and bound molecules are identified by amplifying and identifying (for example by sequencing or by the use of a microarray) the attached DNA sequence. Functional assays may be performed by assessing a known activity of a target in the presence and absence of the test compound.

Preferably, the compound is a (poly)peptide, a nucleic acid molecule, a lipid, a polysaccharide or a small molecule.

The definition of the term "(poly)peptide" as provided herein above also applies *mutatis mutandis* to this embodiment.

"Nucleic acid molecules", in accordance with the present invention, include DNA, such as cDNA or genomic DNA, and RNA. It is understood that the term "RNA" as used herein comprises all forms of RNA including mRNA, ncRNA (non-coding RNA), tRNA and rRNA. The term "non-coding RNA" includes siRNA (small interfering RNA), miRNA (micro RNA), rasiRNA (repeat associated RNA), snoRNA (small nucleolar RNA), and snRNA (small nuclear RNA). Further included are nucleic acid mimicking molecules known in the art such as synthetic or semisynthetic derivatives of DNA or RNA and mixed polymers, both sense and antisense strands. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey (2001) Chem Biol. 8, 1). LNA is an RNA derivative in which the ribose ring is constrained by a methylene linkage between the 2'-oxygen and the 4'-carbon. They may contain additional non-natural or derivatised nucleotide bases, as will be readily appreciated by those skilled in the art.

The term "lipid" as used herein refers to a fatty or waxy organic compound that is readily soluble in nonpolar solvent but not in polar solvent. Lipids are well known in the art and include, for example, waxes, oils, sterols, cholesterol, fat-soluble vitamins, monoglycerides, diglycerides, triglycerides (fats), and phospholipids.

As used herein the term "polysaccharide" refers to a linear or branched carbohydrate molecule composed of monosaccharide units which are joined by glycosidic bonds.

The term "lead compound" as used herein refers to a compound that can be classified as an inhibitor or activator of Hes3 as defined herein above. Such lead compounds are used as starting compounds for developing drugs to treat or prevent diseases characterised by insulin resistance or obesity or to treat or prevent diseases characterised by decreased insulin production, respectively, in that they may be optimised with regard to, e.g., their potency, their pharmacokinetic profile or their suitability to be used in a certain pharmaceutical formulation, so as to arrive at a compound which may be, advantageously, safely and effectively used in a pharmaceutical composition. Methods and tools for the optimisation of the pharmacological properties of compounds identified in screens, the lead compounds, are known in the art. For example, *in silico* tools for optimizing lead compounds are known in the art and described, e.g., in Cruciani et al., European Journal of Pharmaceutical Sciences, vol. 11, suppl. 2, p. S29-S39 (2000). Furthermore, high-throughput approaches for evaluating properties of lead compounds have been described in Tarbit and Berman, Current Opinion in Chemical Biology, vol. 2, issue 3, p. 411-416 (1998).

In a more preferred embodiment of the methods of the invention, the optimisation comprises modifying the lead compound to achieve: i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, uptake into a specific cell type, metabolism and excretion), and/or vii) modified physico-chemical parameters (solubility, hygroscopicity, colour, taste, odour, stability, state), and/or viii) improved general specificity, organ/tissue specificity, and/or ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (I) conversion of alkyl substituents to cyclic analogues, or (m) derivatisation of hydroxyl groups to ketales, acetales, or (n) N-acetylation to amides, phenylcarbamates, or (o) synthesis of Mannich bases, imines, or (p) transformation of ketones or aldehydes to Schiff's bases, oximes, acetales, ketales, enolesters, oxazolidines, thiazolidines or combinations thereof.

The various steps recited above are generally known in the art, as described above. They include or rely on quantitative structure-activity relationship (QSAR) analyses (Kubinyi (1992) "Hausch-Analysis and Related Approaches", VCH Verlag, Weinheim), combinatorial biochemistry, classical chemistry and others (see, for example, Holzgrabe and Bechtold (2000) Deutsche Apotheker Zeitung 140(8), 813).

Further, the present invention also relates to an *in-vitro* or *ex-vivo* method of identifying a compound suitable as a lead compound and/or as a medicament for the treatment and/or prevention of diseases characterised by insulin resistance or of obesity, the method comprising the steps of (a) contacting a test compound with a Hes3 protein, and (b) determining whether said test compound, upon contacting in step (a) inhibits the activity of said Hes3 protein wherein said inhibition indicates a compound suitable as a lead compound and/or as a medicament for the treatment and/or prevention of diseases characterised by insulin resistance or of obesity.

The definitions and preferred embodiments as provided with regard to the use of Hes3 according to the present invention apply *mutatis mutandis* also to this *in-vitro* or *ex-vivo* method. In particular, the Hes3 protein may be an isolated or recombinantly produced protein.

The term *"ex-vivo* or *in-vitro* method" refers to a method that is carried out outside a living organism. In an *ex-vivo* method living cells or tissues that are derived directly from a living organism, i.e. that have not been outside the organism for more than approximately 24 hours, are employed. In addition, an *ex-vivo* method is performed under conditions that resemble the conditions in a living organism, i.e. the *in-vivo* conditions, as closely as possible. By contrast, in an *in-vitro* method molecules, cells or cell lines are employed that have not formed part of a living organism for more than 24 hours, or (in case of molecules) that have never formed part of a living organism because they have been produced synthetically. In addition, an *in-vitro* method may be performed under conditions that do not, or at least not closely, resemble the *in-vivo* conditions.

As used herein the term "contacting" refers to bringing the test compound into such close proximity of the target protein under conditions and for a period of time such that the compound may interact with and/or act on its target protein. At the same time the term encompasses that the conditions such as temperature, pH, under which the contact occurs as well as the concentration of the test compound are such that the contact does not damage the target protein. Suitable conditions and incubation times for ensuring that this is the case are known in the art.

The term "the activity of said Hes3 protein" refers to the ability of the Hes3 protein to bind to interaction partners, to bind to DNA and/or to act as a transcription factor or as a transcriptional repressor. Suitable assays are known in the art and have been described herein above.

In a preferred embodiment, the level of activity is less than 90%, more preferred less than 80%, 70%, 60% or 50% of the activity in absence of the inhibitor. Yet more preferred are inhibitors lowering the level down to less than 25%, less than 10%, less than 5% or less than 1% of the activity in absence of the inhibitor.

It is understood that determining whether a test compound inhibits the activity of Hes3 is only possible once the test compound has been brought into contact with a cell comprising Hes3. Therefore, the steps are performed in the indicated order, i.e. step (a) is performed before step (b).

The analysis whether a test compound inhibits the activity of Hes3 can be performed by using, e.g., high throughput screening assays (HTS). High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to the observed biological activity.

In a preferred embodiment of this *ex-vivo* or *in-vitro* method the Hes3 protein is comprised in a cell.

The "cell" as recited throughout this specification refers to a primary cell or a cell from a cell line. As used herein, the term preferably does not encompass human embryonic stem cells or cells obtained from human embryos. Primary cells are cells which are directly obtained from an organism and which are not immortalized. Suitable primary cells are, for example, cells that are capable of secreting insulin such as pancreatic beta-cells from species such as mouse, rat, human, guinea pig, hamster, pig, dog, sheep, goat, donkey or cow, and are used either in the form of islets of Langerhans or isolated cells. Preferably, the cells are primary pancreatic islet cells from rodents or humans or primary non-dissociated pancreatic islets from rodents and humans. The cell line may be, for example, selected from the rat insulinoma (RIN) cell lines such as INS-1 (Asfari et al. (1992) Endocrinol.130, 167), INS-2 (Asfari et al. (1992) Endocrinol.130, 167), RIN-r (Philippe et al. (1986) Endocrinol. 119, 2833) and RIN-m (Bathena et al. (1982) Diabetes 31, 521; Praz et al. (1983) Biochem. J. 210, 345; Philippe et al. (1987) J. Clin. Invest. 79, 351) or from the hamster insulinoma (HIT) cell lines such as HIT-T15 (Santerre et al. (1981) Proc. Natl. Acad. Sci. U.S.A. 78, 4339) or from mouse beta-cell lines expressing the SV40 large T-antigen (beta-TC lines) such as betaTC1, betaTC2, betaTC3 (Efrat et al. (1988) Proc. Natl. Acad. Sci. U.S.A. 85, 9037), betaTC6 (Poitout et al. (1995) Diabetes 44, 306), betaTC7 (Efrat et al. (1993) Diabetes 42, 901), betaTCtet (Efrat et al. (1995) Proc. Natl. Acad. Sci. U.S.A. 92, 3576) or from mouse insulinoma (MIN) cell lines such as MIN6 (Myazaki et al. (1990) Endocrinol. 127, 126).

As recited hereinabove, said cell comprises a Hes3 protein. Preferably, the protein comprises or consists of the amino acid sequence of any one of SEQ ID NOs:4 to 7 or is encoded by a nucleic acid comprising or consisting of any one of SEQ ID NOs:8 to 10.

With regard to this preferred embodiment the test compound is contacted with the cell comprising the Hes3 protein, i.e. the test compound is brought into such close proximity of the cell under conditions and for a period of time such that the compound may enter the cell and may interact with and/or act on its target protein. In addition, the contacting is performed under conditions that are not toxic or harmful to the cell. Suitable conditions and incubation times for ensuring that this is the case are known in the art.

The activity of Hes3 that can be determined, further to the activities to be assessed when Hes3 is employed as an isolated protein, includes the ability of Hes3 to translocate to the nucleus.

If the test compound is contacted with a cell comprising Hes3, inhibition of Hes3 activity by the test compound may be achieved by the test compound performing one or more of the following effects: (i) the transcription of the gene encoding Hes3 is lowered, i.e. the level of mRNA is lowered, (ii) the translation of the mRNA encoding Hes3 is lowered, (iii) the Hes3 protein performs its biochemical function with lowered efficiency in presence of the inhibitor, and (iv) the Hes3 protein performs its cellular function with lowered efficiency in presence of the inhibitor. Thus, assessing whether a test compound inhibits the activity of Hes3 may involve assessing the amount of Hes3 mRNA, the amount of Hes3 protein and/or the intracellular localisation of Hes3, detecting the binding of Hes3 to its interaction partners or to DNA or assessing its activity as a transcriptional activator or repressor. Suitable assay have been described herein above. Additionally or alternatively it may be determined, whether the test compound reduces or abolishes Hes3-mediated insulin expression and/or secretion.

In addition, the present invention also relates to an in-vitro or ex-vivo method of identifying a compound suitable as a lead compound and/or as a medicament for the treatment and/or prevention of diseases characterised by decreased insulin production, the method comprising the steps of (a) contacting a test compound with a Hes3 protein, and (b) determining whether said test compound, upon contacting in step (a) increases the activity of said Hes3 protein, wherein said increase indicates a compound suitable as a lead compound and/or as a medicament for the treatment and/or prevention of diseases characterised by decreased insulin production.

The definitions and preferred embodiments regarding the terms *"ex-vivo* or *in-vitro* method", "contacting", the Hes3 protein, the term "the activity of said Hes3 protein" and the order of steps as provided with regard to the *in-vitro* or *ex-vivo* method of identifying a compound suitable as a lead compound and/or as a medicament for the treatment and/or prevention of diseases characterised by insulin resistance or of obesity apply *mutatis mutandis* also to this method.

Increasing Hes3 activity refers to an increase of the above defined Hes3 activity.

In a preferred embodiment, the level of activity is increased by more than 10%, such as by more than 25% as compared to the activity in the absence of the test compound. More preferably, the activity is increased by more than 50%, such as by more than 60%, such as by more than 70% such as more than 80%, such as by more than 90% as compared to the activity in the absence of the test compound. Even more preferably, the activity is increased by more than 100% as compared to the activity in the absence of the test compound, i.e. the activity is at least doubled as compared to the activity in absence of the inhibitor. Yet more preferably, the activity is at least tripled as compared to the activity in absence of the inhibitor. Most preferably, the activity is at least 4-fold, such as at least 5-fold, such as at least 10-fold, such as at least 50-fold, such as at least, 80-fold, such as at least 100-fold the activity in absence of the test compounds. Also envisaged is the test compound not only enhancing but eliciting the activity of Hes3. In other words, in absence of the test compound no activity of Hes3 may be observable whereas such an activity can be detected upon addition of the test compound.

In a preferred embodiment of this *ex-vivo* or *in-vitro* method the Hes3 protein is comprised in a cell.

In this embodiment, the test compound may increase the activity of Hes3 by performing one or more of the following effects: (i) the transcription of the gene encoding Hes3 is enhanced, i.e. the level of mRNA is increased, (ii) the translation of the mRNA encoding Hes3 is enhanced, (iii) the Hes3 protein performs its biochemical function with increased efficiency in presence of the inhibitor, and (iv) the Hes3 protein performs its cellular function with increased efficiency in presence of the inhibitor. Thus, assessing whether a test compound increases the activity of Hes3 may involve assessing the amount of Hes3 mRNA, the amount of Hes3 protein and/or the intracellular localisation of Hes3, detecting the binding of Hes3 to its interaction partners or to DNA or assessing its activity as a transcriptional activator or repressor. Suitable assay have been described herein above. Additionally or alternatively it may be determined, whether the test compound elicits or enhances Hes3-mediated insulin expression and/or secretion.

Further, the present invention relates to an in-vitro method of eliciting or enhancing insulin production comprising bringing a cell into contact with Hes3 and/or an activator thereof, wherein said activator is (a) an antibody, an antibody mimetic, a dominant negative protein, an siRNA, an shRNA, an miRNA, a ribozyme, an aptamer, an antisense nucleic acid molecule or a small molecule inhibiting JAK; (b) antibody, an antibody mimetic, a dominant negative protein, an siRNA, an shRNA, an miRNA, a ribozyme, an aptamer, an antisense nucleic acid molecule or a small molecule inhibiting p38; (c) a variant of STAT3 that cannot be phosphorylated on tyrosine 705; (d) a variant of STAT3 that mimics constitutive phosphorylation on serine 727; (e) a soluble variant of Delta4 or jagged 1; (f) an agonistic antibody specifically binding to Notch; or (g) an inhibitor of leptin signalling.

The definitions and preferred embodiments provided herein above apply *mutatis mutandis* also to this *in-vitro* method.

As used herein the term "elicit" refers to inducing an event such as insulin production in a cell where this event was not observable prior to the treatment. Therefore, eliciting insulin production refers to making a cell produce insulin which did not do so prior to the treatment.

The term "enhancing insulin production" refers to increasing the quantity of insulin produced by a cell which already produced insulin albeit in a lesser amount prior to the treatment.

In a preferred embodiment, the amount of insulin produced by the cell is increased by more than 10%, such as by more than 25% as compared to the amount of insulin produced by the cell in the absence of Hes3 and/or the activator thereof. More preferably, the amount of insulin produced by the cell is increased by more than 50%, such as by more than 60%, such as by more than 70% such as more than 80%, such as by more than 90% as compared to the amount of insulin produced by the cell in the absence Hes3 and/or the activator thereof. Even more preferably, the amount of insulin produced by the cell is increased by more than 100% as compared to the amount of insulin produced by the cell in the absence of Hes3 and/or the activator thereof, i.e. the amount of insulin produced by the cell is at least doubled as compared to the activity in absence of Hes3 and/or the activator thereof. Yet more preferably, the amount of insulin produced by the cell is at least tripled as compared to the amount of insulin produced by the cell in absence of Hes3 and/or the activator thereof. Most preferably, the amount of insulin produced by the cell is at least 4-fold, such as at least 5-fold, such as at least 10-fold, such as at least 50-fold, such as at least, 80-fold, such as at least 100-fold the amount of insulin produced by the cell in absence of the Hes3 and/or the activator thereof. Also envisaged is Hes3 and/or the activator thereof not only enhancing but eliciting the production of insulin. In other words, in absence of Hes3 and/or the activator thereof no production of insulin may be observable whereas such a production can be detected upon addition Hes3 and/or the activator thereof.

The cell is a cell capable of producing insulin as defined herein above. Suitable examples of such cells have also been described herein above.

It will be understood that this method relates to the *in-vitro* production of insulin in eukaryotic cells, for example for biotechnological purposes.

In a preferred embodiment of the inhibitor for use according the invention, of the method of treating or preventing a disease characterised by insulin resistance or obesity of the invention, of the method of treating or preventing a disease characterised by decreased insulin production of the invention, of the pharmaceutical composition of the invention, of Hes3 and/or the activator thereof of the invention, of the use of the invention, of the in-vitro method of the invention, or of the in-vitro or ex-vivo method of the invention, said Hes3 protein (i) comprises or consists of the amino acid sequence of any one of SEQ ID NOs: 4 to 7; (ii) is encoded by a nucleic acid molecule comprising or consisting of the sequence of any one of SEQ ID NOs: 8 to 10; (iii) comprises or consists of a fragment of the amino acid sequence of any one of SEQ ID NOs: 4 to 7 and exhibits Hes3 activity; (iv) comprises or consists of a fragment of the Hes3 protein encoded by the nucleic acid molecule of any one of SEQ ID NOs: 8 to 10 and exhibits Hes3 activity; or (v) comprises or consists of a sequence at least 75% identical with (1) the amino acid sequence of any one of SEQ ID NOs: 4 to 7, (2) the Hes3 protein encoded by the nucleic acid molecule of any one of SEQ ID NOs: 8 to 10; or (3) the fragment according to (iii) and exhibits Hes3 activity.

The definitions and preferred embodiments provided herein above apply *mutatis mutandis* also to this embodiment.

As described herein above and as shown in Table 1, SEQ ID NOs:4 to 7 represent Hes3 isoforms from different organisms.

Group (i) comprises Hes3 proteins which either consist of one of the indicated amino acid sequences or which in addition to one of these sequences comprise further amino acid sequences, such as is the case in fusion proteins. These additional amino acid sequences may for example constitute a tag facilitating purification or detection of the fusion protein. Suitable tags and methods for creating fusion proteins are well known in the art.

The proteins of group (ii) are encoded by a nucleic acid molecule consisting of the nucleotide sequence of any one of SEQ ID NOs:8 to 10 or by a nucleic acid molecule comprising but not consisting of one of these nucleotide sequences. Like the corresponding amino acid sequences, the nucleotide sequences of SEQ ID NOs:8 to 10 are also shown in Table 1 herein above.

The term "fragment of the protein" as defined herein in connection with the various proteins refers to a portion of the protein comprising at least the amino acid residues necessary to maintain the biological activity of the protein.

Preferably a fragment of the protein exhibiting Hes3 activity is a portion of the protein comprising at least the amino acid residues of the bHLH domain (amino acids 1 to 49 of SEQ ID NO:4), the WRPW motif (amino acids 175 to 178 of SEQ ID NO:4) and/or the orange domain (amino acids 65 to 99 of SEQ ID NO:4) or the corresponding domains of a Hes3 protein from a different organism. Methods for identifying the position of a specific domain within a protein are known in the art. It is also preferred that the fragment comprises or consists of at least 30, such as at least 40, such as at least 50, such as at least 60, such as at least 70, such as at least 90, such as at least 100, such as at least 120, such as at least 140, such as at least 160, such as at least 180 contiguous amino acids, i.e. the fragment may be a fragment comprising or consisting of a linear chain of exactly the recited number of amino acids but may also be a fragment comprising or consisting of a linear chain of more than the recited number of amino acids. Furthermore, said fragment exhibits at least one aspect of Hes3 activity as described herein above. It is understood that in the *in-vitro* or *ex-vivo* methods the fragment needs to exhibit at least the aspect of Hes3 activity that is employed as a readout. In other words, if the ability of a test compound to inhibit or enhance Hes3 activity is analysed, for example, by determining Hes3 translocation to the nucleus, the fragment needs to have at least the ability to translocate to the nucleus.

Also encompassed by the present invention are sequences that at least exhibit 75% identity with the above-recited protein. Preferably, the identity is between 75% and 98% such as at least 80%, more preferred at least 90%, more preferred 95% and most preferred the identity is at least 98%. Such molecules may be splice forms, homologous molecules from other species, such as orthologues, or mutated sequences from the same species to mention the most prominent examples. To evaluate the identity level between two nucleotide or protein sequences, they can be aligned electronically using suitable computer programs known in the art. Such programs comprise BLAST (Altschul et al. (1990) J. Mol. Biol. 215, 403), variants thereof such as WU-BLAST (Altschul and Gish (1996) Methods Enzymol. 266, 460), FASTA (Pearson and Lipman (1988) Proc. Natl. Acad. Sci. USA 85, 2444) or implementations of the Smith-Waterman algorithm (SSEARCH, Smith and Waterman (1981) J. Mol. Biol., 147, 195). These programs, in addition to providing a pairwise sequence alignment, also report the sequence identity level (usually in percent identity) and the probability for the occurrence of the alignment by chance (P-value). Programs such as CLUSTALW (Higgins et al. (1994) Nucleic Acids Res. 22, 4673) can be used to align more than two sequences.

The considerations regarding Hes3 activity provided with regard to the fragment of groups (iii) and (iv) also apply to the proteins of group (v).

In another preferred embodiment of the inhibitor for use according to the invention, of the method of treating and/or preventing a disease characterised by insulin resistance or obesity of the invention, of the use according to the invention or of the in-vitro or ex-vivo method of identifying a compound suitable as a lead compound and/or as a medicament for the treatment and/or prevention of diseases characterised by insulin resistance or obesity of the invention, the disease characterised by insulin resistance is type II diabetes or metabolic syndrome.

The definitions and preferred embodiments provided herein above apply *mutatis mutandis* also to this embodiment.

In a preferred embodiment of the pharmaceutical composition of the invention, of Hes3 and/or an activator thereof for use according to the invention, of the method of treating and/or preventing a disease characterised by decreased insulin production of the invention, of the use according to the invention, or of the *in-vitro* or *ex-vivo* method of identifying a compound suitable as a lead compound and/or as a medicament for the treatment and/or prevention of diseases characterised by decreased insulin production, the disease characterised by decreased insulin production is type I diabetes.

Type 1 diabetes (previously known as insulin-dependent, juvenile or childhood-onset) is characterized by deficient insulin production. The exact cause of type 1 diabetes is not known but the disease is considered to involve the destruction of the beta cells of the pancreas which is thought to involve an aberrant reaction of the immune system. Symptoms, which may occur suddenly, include excessive excretion of urine (polyuria), thirst (polydipsia), constant hunger, weight loss, vision changes and fatigue.

In a further preferred embodiment of the inhibitor for use according to the invention or of the method of treating and/or preventing a disease characterised by insulin resistance or obesity of the invention, the inhibitor is an antibody, an antibody mimetic, a dominant negative protein, an siRNA, an shRNA, an miRNA, a ribozyme, an aptamer, an antisense nucleic acid molecule or a small molecule.

The definitions and preferred embodiments provided herein above apply *mutatis mutandis* also to this embodiment.

In another preferred embodiment of the pharmaceutical composition of the invention, of Hes3 and/or the activator thereof for use according to the invention, or the method of treating and/or preventing a disease characterised by decreased insulin production of the invention, Hes3 and/or the activator thereof is to be administered to the beta cells of the pancreas.

The definitions and preferred embodiments provided herein above apply *mutatis mutandis* also to this embodiment.

Type 1 diabetes is considered to result mainly from the production of the beta cells of the pancreas. An involvement of other cell types or tissues, if any, is considered to be minor. Therefore, a targeted administration of Hes3 and/or the activator thereof to the remaining beta cells and/or to the pancreas is envisaged in preventing and/or treating diseases characterised by decreased insulin production such as type 1 diabetes. Means and methods for such a localised administration are known in the art and have been described herein above.

As regards the embodiments characterised in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all attached claims.

The figures show:

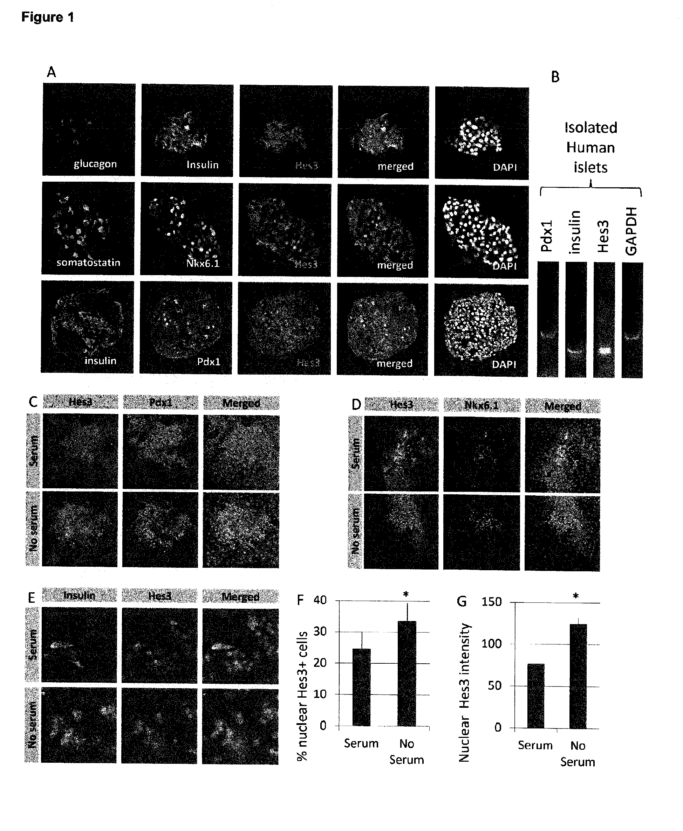

**Figure 1****: Hes3 is expressed in adult human pancreatic islets and dissociated cultures.** (A) Confocal immunohistochemical images of sections from isolated human pancreatic islets show nuclear Hes3 expression in cells co-expressing glucagon, insulin, somatostatin, Nkx6.1, and Pdx1 [Image widths: 246µm]. (B) PCR analysis from isolated human pancreatic islets shows Hes3, Pdx1, and insulin expression. (C-E) Cultures from dissociated human pancreatic islets in serum-free and serum-containing conditions exhibit Hes3 expression; Hes3 co-localizes with Pdx1, Nkx6.1, and insulin [Image widths: 246µm]. (F) Quantification of the percentage of cells with nuclear Hes3 staining in cultures from dissociated human pancreatic islets in the presence or absence of serum. (G) Quantification of nuclear Hes3 signal intensity in cultures from dissociated human pancreatic islets in serum-free and serum-containing conditions [Signal intensity was measured using the Fiji image processing software. Data are means from 500+ cells; error bars represent s.e.m.].

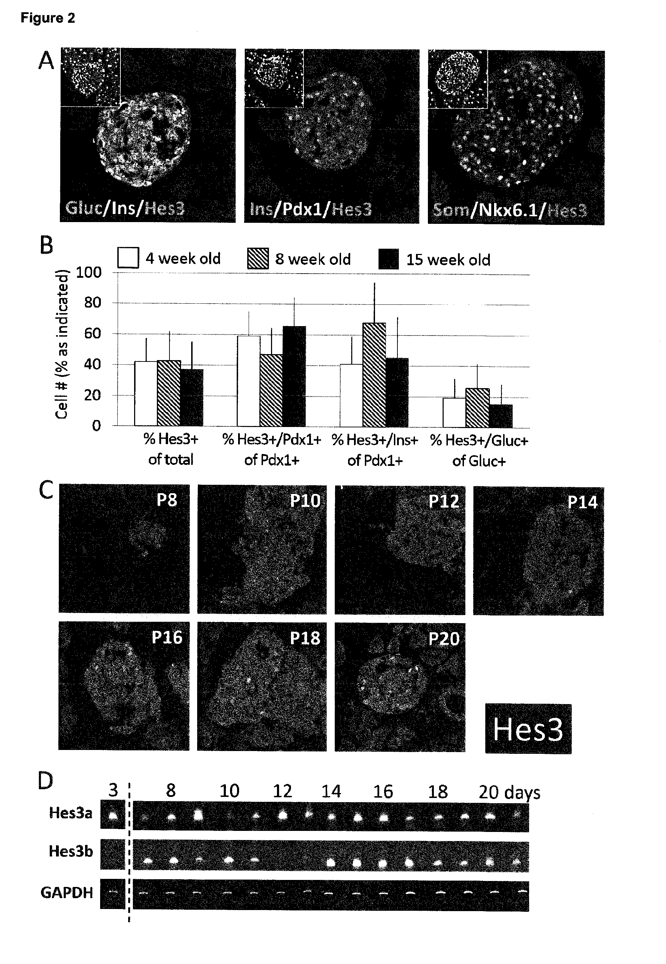

**Figure 2****: Hes3 is expressed in mouse pancreatic islets.** (A) Confocal immunohistochemical images of sections from the adult mouse pancreas show that Hes3 is expressed in the nucleus of pancreatic islet cells co-expressing glucagon, insulin, and Nkx6.1 [Image widths: 246µm; insets show cell nuclei stained with DAPI]. (B) Quantification of the percentage of cells in the islet that express Hes3, the percentage of insulin positive cells in the islet that express Hes3, and the percentage of glucagon positive cells in the islet that express Hes3 in 4, 8, and 15 week old mice [N=3; errors bars represent s.e.m.s]. (C) Immunohistochemical images of sections from the adult mouse pancreas show that Hes3 immunoreactivity appears postnatally ["P8": postnatal day 8, etc.]. [Image widths: 246µm]. (D) Hes3 mRNA expression in cDNA from total pancreas from mice of different ages (postnatal days 3 to 20) - (data are shown for both isoforms of Hes3: Hes3a and Hes3b).

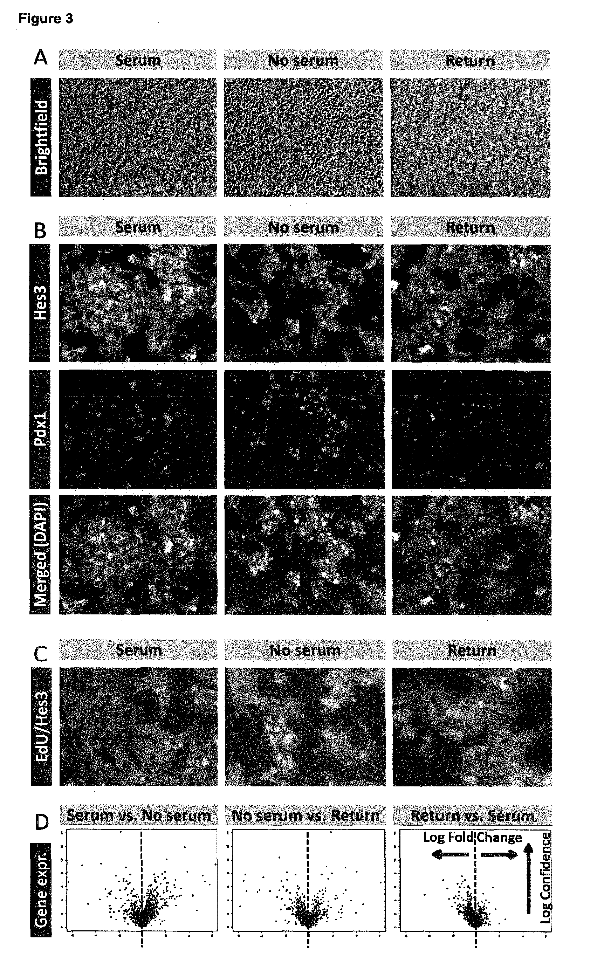

**Figure 3****: Hes3 is expressed in cultured MIN6 cells under defined conditions.** (A) Cells cultured in all three conditions grow efficiently (representative brightfield images shown) [Image widths: 450µm]. (B) Serum-free conditions promote nuclear Hes3 localization and increase nuclear Pdx1 localization. Nuclear Hes3 and Pdx1 co-localize. (Representative immunocytochemistry images shown) [Image widths: 450µm]. (C) Cells cultured in all three conditions incorporate EdU (representative immunocytochemistry images shown) [image width: 125µm]. (D) Culture conditions affect gene expression profiles as revealed by Affymetrix DNA microarrays. Dots left of the vertical dotted line represent downregulated genes and dots right of the vertical dotted line represent upregulated genes.

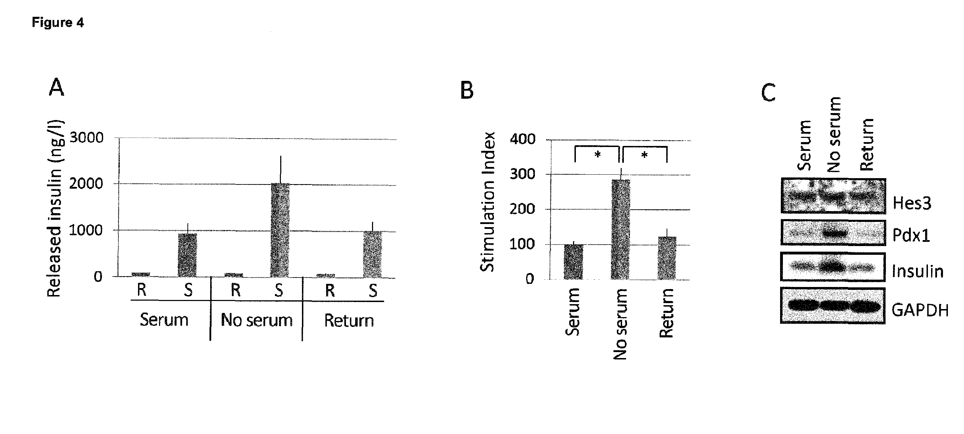

**Figure 4****: MIN6 cells cultured in serum-free conditions respond to glucose challenge and pharmacological stimulation.** (A) Released insulin measurements by ELISA under the three different conditions show that the cells are responsive to glucose challenge in all conditions. [N=3; errors bars represent s.e.m.s. "R": Resting state; "S": Stimulated state]. (B) Stimulation index measurements (expressed as % of the value in serum-containing conditions) by ELISA under the three different conditions show that the cells are responsive to glucose challenge in all conditions. [N=3; errors bars represent s.e.m.s]. (C) Culture conditions affect Pdx1 expression and insulin content (Western blotting images shown); Serum-free conditions increase Pdx1 expression and insulin content; the effects are reversed by re-plating the cells in RC [GAPDH is used as the housekeeping gene].

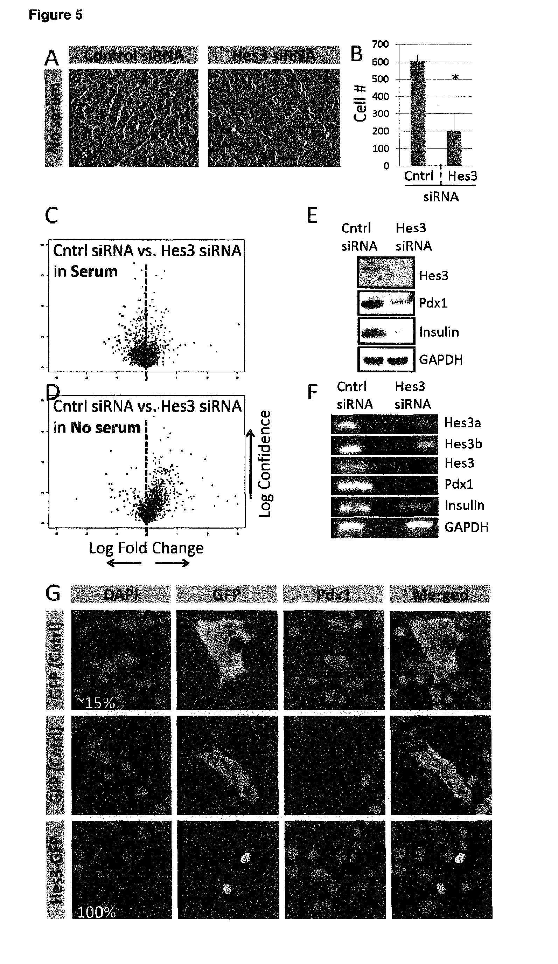

**Figure 5****: Hes3 regulates cell number and gene expression in MIN6 cell cultures.** (A) Hes3 interference by siRNA opposes their growth in serum-containing conditions. (Representative brightfield images shown) [Image width: 450µm]. (B) Quantification of cell number following Hes3 interference in serum-free conditions. Data are presented as number of cells per field of view. [N=3; errors bars represent s.d.]. (C,D) Hes3 interference by siRNA in cells cultured in serum-containing and serum-free conditions induces gene expression profile changes (5 days after interference). Data are presented as a "Volcano" plot generated from Affymetrix DNA microarrays. The y axis represents statistical confidence (from quadruplicate samples) and the x axis represents fold change (logarithmic scale). Dots left of the vertical dotted line represent genes whose expression is reduced by Hes3 siRNA and dots right of the vertical dotted line represent those whose expression increases. (E) Hes3 interference opposes Hes3, Pdx1, and insulin expression. (Western blotting data shown; GAPDH was used as the housekeeping gene). (F) Hes3 interference opposes Pdx1 and insulin expression. (PCR data shown; GAPDH was used as the housekeeping gene). (G) Hes3 overexpression induces nuclear Pdx1 localization in DC. In DC, at each time point, approximately 15% of cells exhibit nuclear Hes3 localization. Transient overexpression of a control GFP plasmid has no effect on nuclear Pdx1 localization. The top two rows show examples of GFP-labelled cells with nuclear and non-nuclear Pdx1. In contrast, overexpression of a Hes3-GFP fusion plasmid results in 100% of labelled cells exhibiting nuclear Pdx1. (Representative immunocytochemistry images shown) [image width: 246µm].

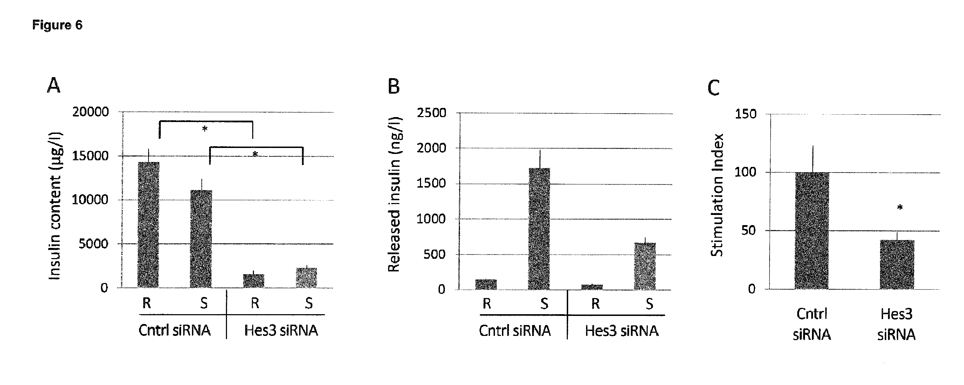

**Figure 6****: Hes3 regulates responses to glucose challenge in MIN6 cell cultures.** (A-C) Insulin content, released insulin, and stimulation index (expressed as % of control siRNA) measurements by ELISA following transfection with either a control siRNA or Hes3 siRNA show that opposing Hes3 expression reduces the responsiveness to glucose challenge in MIN6 cells cultured in serum-free conditions. [N=3; errors bars represent s.e.m. "R": Resting state; "S": Stimulated state].
**Figure 7****: Hes3 null mice exhibit a more pronounced effect following STZ-induced damage.** (A) Immunohistochemical detection of pancreatic islet markers in sections from whole pancreas [Image widths: 246µm]. (B) PCR analysis of Hes1 expression in isolated islets from wild type and Hes3 mice. (C) X-Gal staining in Hes3 null mice following damage by the low STZ protocol. (D) X-Gal staining in Hes3 null mice following damage by the high STZ protocol. (E) Blood glucose level progression after damage by the low STZ protocol in wild type and Hes3 null mice. (F) Immunohistochemical detection of Nkx6.1 in sections from total pancreas in wild type and Hes3 null mice 5 days after damage by the low STZ protocol.]
**Figure 8****: Hes3 null mice are more resistant to weight gain and diabetes induced by high fat diet (HFD).**
   (A) Normal mice placed on a high fat diet (11 weeks on HFD) gain weight. This effect is much less pronounced in mice in which Hes3 has been knocked out (Hes3 null mice). (B) Wild type mice develop type 2 diabetes within a few (11) weeks of being placed on a HFD as is shown by their inability to efficiently reduce blood glucose levels (as determined by a glucose tolerance test). By contrast Hes3 null mice can efficiently reduce glucose levels even on a HFD and do not develop type 2 diabetes.
**Figure 9****: Induction of Hes3 expression outside the pancreatic islet.** Immunohistochemical stainings of perfused and fixed mouse pancreata sections for Hes3 and insulin (and DAPI, as a cell nucleus marker). Normal islet exhibit Hes3 staining in the islets but not outside of the islets. In contrast, islets from db/db, ob/ob, and irradiated wild-type mice exhibit Hes3 staining outside of the islets. Specifically, Hes3 staining is seen associate with the pancreatic duct; Hes3+ cells also express insulin. [Irradiation regimen: a single 4 Gray dose of irradiation for 3 minutes and 41 seconds; pancreata were extracted 5 days after irradiation].

The examples illustrate the invention

### Example 1: Material and Methods

*Cell culture -* MIN6 were grown in either serum containing medium (DMEM (Life Technologies, 61965-026), 15% Fetal Calf Serum (Biochrom Superior, F0615), 70 µM 2-Mercaptoethanol (Sigma Aldrich, M6250), 100 µg/mL Pen-Strep (Life Technologies, 15140-122)) or serum free N2 medium (DMEM-F12 (Sigma Aldrich, D8062), 100 µg/mL Apo-Transferrin (Sigma Aldrich, T2036), 20 nM Progesterone (Sigma Aldrich, P8783), 100 µM Putrescine (Sigma Aldrich, P5780), 30 nM Selenite (Sigma Aldrich, S5261), 100 µg/mL Pen-Strep)). To examine changes in the properties of MIN6 under different culture conditions, cells were first maintained in serum containing medium for 5 days. Cells were then passaged and grown under serum free conditions for another 5 days. The cells were then passaged again, back into serum containing medium ("RC" for Return Condition) for an additional 5 days prior to analysis. Serum and no serum controls were run in parallel in which the cells were maintained in either serum containing or serum free conditions for the final 10 days of the experiment. For Exendin-4 treatments, cells were seeded at 50000 cells per 24 well-plate well and treated with 200 nM Exendin-4 (Biotrend, BP0111) beginning at 24 hours after plating. Exendin-4 was added every two days during medium changes, and cells were fixed and immunostained at day 5. Serum-containing medium is denoted as "CC" (Common Conditions) and serum-free medium as "DC" (Defined Conditions).

*DNA Microarray -* RNA was extracted from MIN6 cells grown in T75 flask under the above mentioned culture conditions. For experiments using HES3 siRNA, cells were transfected at 24 hours after seeding. RNA Integrity Number (RIN) for each sample was checked using Agilent Bioanalyzer (Agilent Michigan). Triplicates for each culture conditions were run in DNA microarray equipment (Affymetrix). Volcano plots, heat maps, and statistics were generated using the R-2.15.2 software.

*RNA Isolation and Reverse Transcriptase PCR -* RNA was extracted from whole pancreas or MIN6 cells using High Pure RNA isolation kit (Roche, 11828665001) and reverse transcribed using Promega M-MLV reverse transcriptase (Promega, M170B). PCR was performed for HES3a, HES3b, Pdx1, Insulin and GAPDH. The following primers were used using Dream Taq Green DNA polymerase (Thermo Scientific, EP0711):

**Table 2: Primers.**

| Gene | forward primer | reverse primer |
|---|---|---|
| Mouse HES3a | AAGCTCCCTGCCATAGCGGA | ATGCGTGCACGGCGCTTCTT |
| Mouse HES3b | ACATCACAGCATGGGCACCGAGCCCACATC | GTTGATGCGTGCACGGCGCTTCTTC |
| Mouse HES3 | ATCTCCAAGCCTCTGATGGAGAA | AGCTTTCGTTTCCGTATCTGATG |
| Human Hes3 | GAGAAGCCTTCAGAACTCCTTGC | CTGCCGACCTCATCTCCGCG |
| Mouse Pdx1 | CCACCCCAGTTTACAAGCTC | TGTAGGCAGTACGGGTCCTC |
| Human Pdx1 | CTTTCTCCTCCTCCTCCTTCTA | GGTCATACTGGCTCGTGAATAG |
| Mouse Insulin | GACCAGCTATAATCAGAGACC | AGTTGCAGTAGTTCTCCAGCTG |
| Human Insulin | ATCACTGTCCTTCTGCCA | GGGTGTGTAGAAGAAGCC |
| Mouse GAPDH | CTGGAGAAACCTGCCAAGTA | TGTTGCTGTAGCCGTATTCA |
| Human beta actin | GCCGTCTTGCCCTCCATCGTG | GGAGCCACACGCAGCTCATTGTA |

*Western Blotting -* MIN6 cells were grown in 6-well plates for 5 days, then lysed with 500 µl CytoBuster Protein Extraction Reagent (Novagen, 71009-3) contain protease inhibitors (Sigma Aldrich, P8340). Samples were resolved by western blot using standard techniques. To measure total Hes3, Pdx1, Insulin and house-keeping gene GAPDH in MIN6 and 1.1 B4 cell lines, cells were grown in 6-well plate for 5 days, lysed in 500 µl CytoBuster Protein Extraction Reagent (Novagen, Cat Nr: 71009-3) with 5 µl Protein Inhibitor Cocktail (Sigma Aldrich, P8340) on ice for 30 min and centrifuged for 15 min at 20,000 rpm. The supernatants were collected and protein concentrations were determined using Nanodrops and Qubit Protein Assay Kit (Lifetechnologies). 5 ug of protein was mixed with NuPAGE LDS sample buffer (Life technologies NP0007) and NuPAGE sample reduction agent (Life Technologies NP0009) and boiled at 95 C for 5 min. Samples were loaded in 10% NuPAGE Bis-Tris-Gel (WG1201A) and run for 35 min. Proteins resolved were transferred to a nitrocellulose membrane (iBlot Gel Transfer Stacks, Life Technologies IB301001). The membrane was blocked in 5% Milk and 0.1% Tween 20 in 1xPBS at room temperature for 30 min and incubated with polyclonal HES3 antibody 1:1000 (Santa Cruz, M-135, sc-25393), Anti- Pdx1 antibody 1:10.000 (Abcam,ab47383), Insulin 1:5000 (Santa Cruz, H-86,sc-9168) and Polyclonal anti-GAPDH 1:10.000 (R&D,AF5718) at 4C overnight in rocking platform. Primary antibodies were detected using donkey anti-goat IgG (H+L)-HRPO (Dianova, 805-035-180), goat anti-rabbit IgG (H+L)-HRPO (Dianova, 111-035-144), goat anti-mouse IgG (H+L)-HRPO (Dianova, 115-035-146). All secondary antibodies were diluted 1:5000 in blocking buffer and incubated at RT for 1 hour. Immunoreactive bands was developed using SuperSignal West Pico Chemiluminescent Substrate (Thermoscientific, 34078) and detected by LAS3000 bioimager (FUJI,Japan)

*Immunohistochemistry*/*Immunocytochemistry -* Perfused pancreata were fixed in in 4% PFA at 4°C overnight, dehydrated in 30% Sucrose, embedded in Tissue-Tek-OCT compound (sakura, 4583) and cryo-sectioned (LEICA Germany). 12-14 µm sections were cut and mounted on glass slides. Cells were fixed in 4% PFA at 4°C for 30 min. The sections and cells were permeabilised in 0.1% Triton X-100 in PBS, blocked in 5% Milk and 0.1% Triton X-100 in PBS and incubated overnight at 4°C with anti-HES3 1:100 (Santa Cruz, M-135, sc-25393), Anti- Pdx1 1:1000 (Abcam,ab47383), Anti Insulin 1:200 (GeneTex, GT X27842), Anti-Glucagon 1:200 (GeneTex, K79bB10), Anti-Somatostatin 1:200 (GeneTex, YC7) and anti-NKX6.1 1:200 (rnd systems, AF5857) diluted in blocking buffer. Primary antibodies were detected by incubating the sections with donkey anti-rabbit IgG(H+L) 594 (Dianova,711-505-152), donkey anti-goat IgG (H+L) 488 (Dianova, 705-486-147), donkey anti-guinea pig IgG(H+L) 488 (Dianova, 706-486-148),donkey anti-guinea pig IgG (H+L) 649 (Dianova, 706-496-148), donkey anti-mouse IgG (H+L) 649 (Dianova, 715-496-150 and donkey anti-rat IgG (H+L) 649 (Dianova, 712-496-150). All secondary antibodies were diluted 1:500 in blocking buffer and incubated at room temperature for 1-2 hours. DAPI is applied at 1:10000 dilution for 15 min for nuclear staining. Images were taken using a laser confocal microscope (LEICA,Germany).

*Cell Proliferation -* MIN6 proliferation was determined 4 days after the final cell passage by incubating the cells with 10 µM EdU for 8 hours followed by visualization using the Click-IT EdU Alexa Fluor 594 Imaging Kit (Invitrogen C10339).

*Insulin secretion assay -* Combined glucose/KCI stimulated insulin production was performed on MIN6 cells as described in (Ort, et al. (2001) Embo J 20, 4013-4023) using a Rat Insulin ELISA kit (Mercodia, 10-1250-01) and detected with a microplate reader (TECAN).

*HES3 Knock Down and overexpression -* MIN6 cells were plated at 50000 cells on a 24-well plate. 24 hours later, cells were transfected with HES3 siRNA or scrambled control siRNA (Santa Cruz, sc-37942, sc-37007) using DharmaFECT4 (Thermo Scientific, T-2004-001) as described by the manufacturer. Cells were assayed 12-24 hours post-transfection for changes in proliferation, as well as gene expression as determined by gene array analysis, immunocytochemistry, and western blot. For HES3 overexpression, MIN6 cells were transfected using Lipofectamine 2000 as described by the manufacturer with either pcDNA3.1 containing a C-terminus GFP-tagged HES3a gene or empty vector. Cells were cultured up to day 5, immunostained, and imaged using laser confocal microscopy.

*Mouse models of diabetes -* For high dose Streptozotocin experiments, 8 weeks-old C57BI/6J mice were injected intraperitoneally with a single dose of 150 mg/kg Streptozotocin or PBS (vehicle control). For low dose Streptozotocin experiments, 8 weeks-old C57BI/6J mice were injected intraperitoneally with a 5 daily doses of 50 mg/kg mg/kg Streptozotocin or PBS (vehicle control). Mice were kept for the next 4 weeks before pancreata extraction.

*Db*/*db and Ob*/*ob mice -* Pancreata were harvested from 10 month old mice homozygous for the diabetes spontaneous mutations, Lepr db or Lep ob. C57BI/6J mice served as control.

*Total-body irradiation of mice -* 8 weeks-old C57BI/6J mice were administered a single 4 Gray dose of irradiation for 3 minutes and 41 seconds. Pancreata were extracted 5 days after irradiation.

*In vivo Glucose Tolerance Test (GTT) -* Mice were fasted for 6-8 hours prior to the experiment. Glucose levels were measured 0, 30, 60, 90, and 120 minutes after injection of a 20% glucose solution (25 µl of glucose solution per gram of body weight).

### GTT of mice on HFD

Mice were fasted for 16 hours (overnight). The next day animals were weighed and a first glucose measurement (point 0 for IPGTT) was taken by nicking the tail of each mouse and gently massaging a small drop of blood onto a glucometer strip. A 20% D-glucose stock in PBS was prepared and the volume of the solution to be injected was determined by multiplying the weight of the animal in kg by 10. The solution was injected intraperitoneally (ip) 2 gr/kg of body weight using a 29G x ½ in needle attached to a 1 ml syringe. Glucose measurements were taken at 15, 30, 60, 90, 120 min bas described above. During the assay, animals were provided only with water.

*Human pancreatic islets -* Human isolated islets were obtained from the Diabetes Research Group, King's College London, UK.

*Statistical analysis -* Results shown are the mean ± S.D. or s.e.m.s, as noted in the figure legends. Asterisks identify experimental groups that were significantly different (p-value, 0.05) from control groups by the Student's t-test (paired, two-sided) (Microsoft Excel), where applicable.

### Example 2: Hes3 is expressed in adult human pancreatic islets

Hes3 immunolabelling of isolated adult human pancreatic islets shows expression of Hes3 (counter-stained for insulin, Pdx1, Nkx6.1, glucagon, and somatostatin) (Fig. 1A). PCR analysis confirmed Hes3 expression (Fig. 1B). Isolated adult human pancreatic islets can be placed in culture for research or transplantation purposes (Ludwig, et al. (2013) Proc Natl Acad Sci U S A 110,19054-19058). Typically, these cells are cultured in serum-containing media. However, in NSC cultures, serum inclusion in the culture medium opposes the nuclear localization of Hes3, and this may suppress transcriptional functions (Poser et al. (2013) Front Physiol 4, 273). For this purpose, dissociated human islet cells were cultured in both commonly used serum-containing ("CC") and serum-free defined ("DC") conditions. As detailed above, these conditions differ not only in terms of the presence or absence of serum but also in other aspects. Under both conditions, cells grew efficiently and the cultures maintained expression of Pdx1, Nkx6.1, and insulin (Fig. 1 C-E). The incidence of cells with nuclear Hes3 and the Hes3 label intensity was higher in DC than in CC (Fig. 1F, G).

### Example 3: Hes3 is expressed in mouse pancreatic islets

Hes3 immunolabelling of the adult mouse pancreas shows that Hes3 is expressed in a significant proportion of beta and alpha cells (Fig. 2A,B). Insets are images of the cell nuclei stained with DAPI. Hes3 immunofluorescence appeared postnatally, at approximately day 8 and gradually increased until approximately day 20 (Fig. 2C). Insets show merged channel images. PCR analysis of total adult mouse pancreas confirmed the expression of both Hes3 isoforms (Hes3a and Hes3b) (Fig. 2D).

### Example 4: Hes3 is expressed in cultured MIN6 cells under defined conditions

The mouse insulinoma cell line MIN6 allows the assessment of cell growth and glucose-stimulated insulin secretion (Miyazaki et al. (1990) Endocrinology 127, 126-132). MIN6 cells are typically cultured in serum-containing conditions (CC). Here, the cells were additionally cultured in serum-free conditions (DC) to assess whether, like with the human cells, these conditions promote nuclear Hes3 expression. In some experiments, following growth in DC, cells were passaged back in serum-containing conditions (Return Conditions, "RC") to assess the reversibility of the cell state (Fig. 3A). Cells grew efficiently in all three conditions (Fig. 3B). In CC and RC, no cells exhibited nuclear localization of Hes3. In contrast, in DC, approximately 15% of cells exhibited strong nuclear Hes3 localization at each point in time (Fig. 3C). Cells under all conditions incorporated EdU (Fig. 3D) [% of cells that incorporate EdU following an 8h EdU pulse: CC: 47.7% ± 8.4; DC: 15.0% ± 2.1; RC: 49.8% ± 9.7]. In DC, cells also exhibited a higher incidence of Pdx1 expression. Gene expression differed between CC and DC, and between DC and RC, as revealed by DNA microarray analysis (Fig. 3E). CC and RC conditions were similar.

### Example 5: MIN6 cells cultured in serum-free conditions respond to glucose challenge and pharmacological stimulation

Cells in all three conditions were subjected to a period of glucose starvation followed by a challenge with a combination of glucose and KCI as described previously (Ort, T., et al. (2001) Embo J 20, 4013-4023). Under all conditions cells responded by releasing insulin (Fig. 4A,B). In DC, the amount of released insulin and the stimulation index were elevated compared to CC and RC conditions. In DC, protein expression of Pdx1 and insulin were higher than in CC or RC (Fig. 4C). Total Hes3 expression was similar in all three conditions.

### Example 6: Hes3 regulates cell number and gene expression in MIN6 cell cultures

Hes3 RNA interference reduced cell number in DC; in contrast, it had no significant effect in CC (Fig. 5A,B). Hes3 RNA interference in DC induced significant changes in gene expression as revealed by an Affymetrix DNA microarray experiment; Hes3 knockdown in CC had a smaller effect on gene expression (Fig. 5C,D; Table 2).

**Table 3: Gene expression regulation by Hes3 knockdown in MIN6 cells. MIN6 cells were passaged into separate plates and cultured in CC or DC for 1 day prior to transfection. Cells were transfected with scrambled control siRNA orHes3 siRNA, and total RNA was collected after 4 days in vitro. Gene expression profiles were determined using an Affymetrix DNA microarray. [Data are from a single experiment with quadruplicate samples; top 10 log2FC values are presented for each category].**

| **Genes up-regulated by Hes3 siRNA in CC** | | | | **Genes down-regulated by Hes3 siRNA in CC** | | |
|---|---|---|---|---|---|---|
| **Gene** | **log2FC** | **p value** | | **Gene** | **log2FC** | **p value** |
| Rny3 | 1.58 | 0.02 | | Mesdc2 | -1.06 | 0.01 |
| Mfge8 | 0.76 | 0.02 | | Golph3 | -1.14 | 0.05 |
| Mpzl1 | 0.58 | 5.4e-07 | | Cnih4 | -0.79 | 0.0005 |
| Snord32a | 0.84 | 0.01 | | Lclat1 | -0.92 | 0.02 |
| Unc79 | 0.71 | 0.03 | | Ifi30 | -0.71 | 0.02 |
| Snord57 | 0.61 | 0.04 | | Ifnar2 | -0.70 | 0.006 |
| Snord53 | 2.12 | 0.04 | | Cckbr | -0.95 | 0.02 |
| Pspc1 | 0.61 | 0.04 | | Actr10 | -0.73 | 0.003 |
| Jnc80 | 0.76 | 0.004 | | Nrsn1 | -0.82 | 0.002 |
| Snord35a | 3.05 | 0.002 | | Tuba1a | -0.65 | 0.02 |

| **Genes up-regulated by Hes3 siRNA in DC** | | | | **Genes down-regulated by Hes3 siRNA in DC** | | |
|---|---|---|---|---|---|---|
| **Gene** | **log2FC** | **p value** | | **Gene** | **log2FC** | **p value** |
| Sic25a40 | 0.90 | 0.01 | | Igkv2-137 | -0.90 | 0.009 |
| Cdkn2c | 0.59 | 0.01 | | Gm19684 | -1.18 | 0.003 |
| Pcx | 0.75 | 0.002 | | Rbm3 | -0.78 | 0.04 |
| Fam171b | 1.47 | 3.1e-05 | | Olfr1371 | -1.36 | 0.0006 |
| Gm10632 | 0.82 | 0.0002 | | Sod1 | -0.92 | 0.02 |
| 0610031J06 Rik | 0.74 | 0.0007 | | Sst | -0.66 | 0.03 |
| Nefl | 0.83 | 0.002 | | Olfr331 | -1.23 | 0.005 |
| Scd1 | 0.90 | 0.008 | | Ighv1-83 | -1.09 | 0.02 |
| Pcdhga10 | 1.33 | 0.007 | | Ifna5 | -1.20 | 0.02 |
| Emc2 | 0.75 | 0.03 | | Trav4-2 | -0.68 | 0.0006 |

Hes3 RNA interference reduced the expression of Pdx1 and insulin in DC as assessed by Western blotting and PCR approaches (Fig. 5E,F). Transient overexpression with a control vector with the GFP gene and a vector with the GFP gene fused to Hes3 revealed that all (100%) of the cells transfected with Hes3-GFP also exhibited Pdx1 expression. In contrast, cells transfected with the control GFP vector did not have a greater incidence of Pdx1 expression than non-transfected control (∼15%) (Fig. 5G).

### Example 7: Hes3 regulates responses to glucose challenge in MIN6 cell cultures

Hes3 RNA interference reduced insulin content, the amount of released insulin, and stimulation index as assessed by ELISA measurements of insulin (Fig. 6A-C). The stimulation index is a measure of the ability of insulin-producing cells to release insulin when stimulated. Different stimulation protocols can be used. Here a combination of glucose and potassium Chloride (KCI) which promotes cells to release insulin was used. Released insulin was measured by ELISA.

### Example 8: Lack of Hes3 renders mice more susceptible to Streptozotocin (STZ)-induced damage in a model for type 1 diabetes

Hes3 null mice have no obvious phenotype under normal conditions. Indeed, islet morphology and biomarker expression did not reveal any obvious differences from wild type mice (Fig. 7A). Fasting blood glucose levels and glucose tolerance test (GTT) scores were normal (Fig. 7B, C). Following STZ damage (using either high or low dose STZ protocols), however, activation of the Hes3a promoter was significantly enhanced, as assessed by X-Gal staining (Fig. 7D, E), suggesting a role of Hes3 in this damage paradigm. When wild type mice are subjected to the low STZ damage protocol, blood glucose levels increase approximately 3 days after the last STZ injection, relative to vehicle-treated animals (Fig. 7 F). In contrast, blood glucose levels in Hes3 null mice increased within one day and reached higher levels than wild-types. Immunohistochemical analysis of the pancreata of wild type and Hes3 null mice 5 days after the last STZ injection revealed no obvious effect of glucagon+ alpha cells between the two mouse strains, but a significant reduction in beta cell number as assessed by counting insulin+, Pdx1+, and Nkx6.1+ cells (Fig. 7G). This indicates that Hes3 is required in the protection and/or regeneration from STZ induced damage. Treatment with STZ selectively damages the pancreatic beta cells and is therefore an established model for type 1 diabetes. Thus, the above data support a protective role for Hes3 with regard to type 1 diabetes.

### Example 9: Hes3 null mice are more resistant to weight gain and diabetes induced by high fat diet, a model for type 2 diabetes.

Hes3 null mice gain much less weight than wild type controls when placed on a high fat diet. Hes3 null mice look very normal to the eye, whereas normal mice are obviously obese. After 10 weeks of HFD wild-type mice on average weigh 13 grams more than Hes3 null mice. In addition, after 11 weeks on a HFD wild-type mice are not able to lower their blood glucose levels as efficiently as the wild-type mice placed on a normal diet (ND). By contrast, HFD-fed Hes3 null mice are capable of reducing their blood glucose levels, just like ND-fed Hes3 null mice. This is indicative of the wild-type mice but not the Hes3 null mice developing type 2 diabetes. Overall, this shows that lack of Hes3 protects from weight gain and type 2 diabetes.

## Claims

1. An inhibitor of Hairy and Enhancer of Split 3 (Hes3) for use in a method of preventing or treating
(i) a disease **characterised by** insulin resistance or
(ii) obesity.

2. A method for preventing or treating
(i) a disease **characterised by** insulin resistance or
(ii) obesity,
comprising administering a therapeutically effective amount of an inhibitor of Hes3 to a subject in need thereof.

3. The inhibitor for use according to claim 1, or the method of claim 2, wherein the inhibitor of Hes3 is to be used
(a) as the sole pharmaceutically active ingredient; or
(b) in combination with a further therapeutic agent, preferably a further therapeutic agent for the same medical indication.

4. A pharmaceutical composition comprising Hes3.

5. Hes3 and/or an activator thereof for use in a method of preventing or treating a disease **characterised by** decreased insulin production wherein said activator of Hes3 is
(a) an antibody, an antibody mimetic, a dominant negative protein, an siRNA, an shRNA, an miRNA, a ribozyme, an aptamer, an antisense nucleic acid molecule or a small molecule inhibiting Janus protein tyrosine kinase (JAK);
(b) an antibody, an antibody mimetic, a dominant negative protein, an siRNA, an shRNA, an miRNA, a ribozyme, an aptamer, an antisense nucleic acid molecule or a small molecule inhibiting p38;
(c) a variant of STAT3 that cannot be phosphorylated on tyrosine 705;
(d) a variant of STAT3 that mimics constitutive phosphorylation on serine 727;
(e) a soluble variant of Delta4 or jagged 1;
(f) an agonistic antibody specifically binding to Notch; or
(g) an inhibitor of leptin signalling.

6. A method for preventing or treating a disease **characterised by** decreased insulin production comprising administering a therapeutically effective amount of Hes3 and/or an activator thereof to a subject in need thereof, wherein said activator of Hes3 is
(a) an antibody, an antibody mimetic, a dominant negative protein, an siRNA, an shRNA, an miRNA, a ribozyme, an aptamer, an antisense nucleic acid molecule or a small molecule inhibiting JAK;
(b) an antibody, an antibody mimetic, a dominant negative protein, an siRNA, an shRNA, an miRNA, a ribozyme, an aptamer, an antisense nucleic acid molecule or a small molecule inhibiting p38;
(c) a variant of STAT3 that cannot be phosphorylated on tyrosine 705;
(d) a variant of STAT3 that mimics constitutive phosphorylation on serine 727;
(e) a soluble variant of Delta4 or jagged 1;
(f) an agonistic antibody specifically binding to Notch; or
(g) an inhibitor of leptin signalling.

7. The pharmaceutical composition of claim 4, Hes3 and/or the activator thereof for use according to claim 5, or the method of claim 6, wherein Hes3 and/or the activator thereof is/are to be used
(a) as the sole pharmaceutically active ingredient; or
(b) in combination with a further therapeutic agent.

8. Use of Hes3 as a target in a screen for identifying a compound suitable as a lead compound and/or as a medicament for the treatment and/or prevention of
(a) diseases **characterised by** insulin resistance or of obesity; or
(b) diseases **characterised by** decreased insulin production.

9. An in-vitro or ex-vivo method of identifying a compound suitable as a lead compound and/or as a medicament for the treatment and/or prevention of diseases **characterised by** insulin resistance or of obesity, the method comprising the steps of
(a) contacting a test compound with a Hes3 protein, and
(b) determining whether said test compound, upon contacting in step (a) inhibits the activity of said Hes3 protein
wherein said inhibition indicates a compound suitable as a lead compound and/or as a medicament for the treatment and/or prevention of diseases **characterised by** insulin resistance or of obesity.

10. An in-vitro or ex-vivo method of identifying a compound suitable as a lead compound and/or as a medicament for the treatment and/or prevention of diseases **characterised by** decreased insulin production, the method comprising the steps of
(a) contacting a test compound with a Hes3 protein, and
(b) determining whether said test compound, upon contacting in step (a) increases the activity of said Hes3 protein
wherein said increase indicates a compound suitable as a lead compound and/or as a medicament for the treatment and/or prevention of diseases **characterised by** decreased insulin production.

11. An in-vitro method of eliciting or enhancing insulin production comprising bringing a cell into contact with Hes3 and/or an activator thereof, wherein said activator is
(a) an antibody, an antibody mimetic, a dominant negative protein, an siRNA, an shRNA, an miRNA, a ribozyme, an aptamer, an antisense nucleic acid molecule or a small molecule inhibiting JAK;
(b) an antibody, an antibody mimetic, a dominant negative protein, an siRNA, an shRNA, an miRNA, a ribozyme, an aptamer, an antisense nucleic acid molecule or a small molecule inhibiting p38;
(c) a variant of STAT3 that cannot be phosphorylated on tyrosine 705;
(d) a variant of STAT3 that mimics constitutive phosphorylation on serine 727;
(e) a soluble variant of Delta4 or jagged 1;
(f) an agonistic antibody specifically binding to Notch; or
(g) an inhibitor of leptin signalling.

12. The inhibitor for use according to claim 1 or 3, the method of claim 2, 3, 6 or 7, the pharmaceutical composition of claim 4 or 7, Hes3 and/or the activator thereof of claim 5 or 7, the use of claim 8, the in-vitro method of claim 11, or the in-vitro or ex-vivo method of claim 9 or 10, wherein said Hes3 protein
(i) comprises or consists of the amino acid sequence of any one of SEQ ID NOs:4 to 7;
(ii) is encoded by a nucleic acid molecule comprising or consisting of the sequence of any one of SEQ ID NOs:8 to 10;
(iii) comprises or consists of a fragment of the amino acid sequence of any one of SEQ ID NOs:4 to 7 and exhibits Hes3 activity;
(iv) comprises or consists of a fragment of the Hes3 protein encoded by the nucleic acid molecule of any one of SEQ ID NOs:8 to 10 and exhibits Hes3 activity; or
(v) comprises or consists of a sequence at least 75% identical with
(1) the amino acid sequence of any one of SEQ ID NOs:4 to 7;
(2) the Hes3 protein encoded by the nucleic acid molecule of any one of SEQ ID NOs:8 to 10; or
(3) the fragment according to (iii)
and exhibits Hes3 activity.

13. The inhibitor for use according to claim 1, 3 or 12, the method of claim 2, 3 or 12, the use of claim 8, or the in-vitro or ex-vivo method of claim 9 or 12, wherein the disease **characterised by** insulin resistance is type II diabetes or metabolic syndrome.

14. The pharmaceutical composition of claim 4, 7 or 12, Hes3 and/or the activator thereof for use according to claim 5, 7 or 12, the method of claim 6, 7 or 12, the use of claim 8, or the in-vitro or ex-vivo method of any one of claims claim 10 or 12, wherein the disease **characterised by** decreased insulin production is type I diabetes.

15. The inhibitor of any one of claims 1, 3, 12 or 13 or the method of any one of claims 2, 3, 12 or 13 wherein the inhibitor is an antibody, an antibody mimetic, a dominant negative protein, an siRNA, an shRNA, an miRNA, a ribozyme, an aptamer, an antisense nucleic acid molecule or a small molecule.

16. The pharmaceutical composition of claim 4, 7, 12 or 14, Hes3 and/or the activator thereof for use according to claim 5, 7, 12 or 14, or the method of claim 6, 7, 12 or 13, wherein Hes3 and/or the activator thereof is to be administered to the beta cells of the pancreas.


SEQUENCE LISTING

<110> Technische Universität Dresden

<120> Manipulation of hairy and enhancer of split 3 (Hes3) and its
regulators/mediators in diabetes, obesity, and metabolic syndrome

<130> X3130 EP

<160> 10

<170> BiSSAP 1.3

<210> 1
<211> 770
<212> PRT
<213> Homo sapiens

<400> 1

<210> 2
<211> 770
<212> PRT
<213> Mus musculus

<400> 2
Met Ala Gln Trp Asn Gln Leu Gln Gln Leu Asp Thr Arg Tyr Leu Glu
1               5                   10                  15
Gln Leu His Gln Leu Tyr Ser Asp Ser Phe Pro Met Glu Leu Arg Gln
            20                  25                  30
Phe Leu Ala Pro Trp Ile Glu Ser Gln Asp Trp Ala Tyr Ala Ala Ser
        35                  40                  45
Lys Glu Ser His Ala Thr Leu Val Phe His Asn Leu Leu Gly Glu Ile
    50                  55                  60
Asp Gln Gln Tyr Ser Arg Phe Leu Gln Glu Ser Asn Val Leu Tyr Gln
65                  70                  75                  80
His Asn Leu Arg Arg Ile Lys Gln Phe Leu Gln Ser Arg Tyr Leu Glu
                85                  90                  95
Lys Pro Met Glu Ile Ala Arg Ile Val Ala Arg Cys Leu Trp Glu Glu
            100                 105                 110

Ser Arg Leu Leu Gln Thr Ala Ala Thr Ala Ala Gln Gln Gly Gly Gln
        115                 120                 125
Ala Asn His Pro Thr Ala Ala Val Val Thr Glu Lys Gln Gln Met Leu
    130                 135                 140
Glu Gln His Leu Gln Asp Val Arg Lys Arg Val Gln Asp Leu Glu Gln
145                 150                 155                 160
Lys Met Lys Val Val Glu Asn Leu Gln Asp Asp Phe Asp Phe Asn Tyr
                165                 170                 175
Lys Thr Leu Lys Ser Gln Gly Asp Met Gln Asp Leu Asn Gly Asn Asn
            180                 185                 190
Gln Ser Val Thr Arg Gln Lys Met Gln Gln Leu Glu Gln Met Leu Thr
        195                 200                 205
Ala Leu Asp Gln Met Arg Arg Ser Ile Val Ser Glu Leu Ala Gly Leu
    210                 215                 220
Leu Ser Ala Met Glu Tyr Val Gln Lys Thr Leu Thr Asp Glu Glu Leu
225                 230                 235                 240
Ala Asp Trp Lys Arg Arg Gln Gln Ile Ala Cys Ile Gly Gly Pro Pro
                245                 250                 255
Asn Ile Cys Leu Asp Arg Leu Glu Asn Trp Ile Thr Ser Leu Ala Glu
            260                 265                 270
Ser Gln Leu Gln Thr Arg Gln Gln Ile Lys Lys Leu Glu Glu Leu Gln
        275                 280                 285
Gln Lys Val Ser Tyr Lys Gly Asp Pro Ile Val Gln His Arg Pro Met
    290                 295                 300
Leu Glu Glu Arg Ile Val Glu Leu Phe Arg Asn Leu Met Lys Ser Ala
305                 310                 315                 320
Phe Val Val Glu Arg Gln Pro Cys Met Pro Met His Pro Asp Arg Pro
                325                 330                 335
Leu Val Ile Lys Thr Gly Val Gln Phe Thr Thr Lys Val Arg Leu Leu
            340                 345                 350
Val Lys Phe Pro Glu Leu Asn Tyr Gln Leu Lys Ile Lys Val Cys Ile
        355                 360                 365
Asp Lys Asp Ser Gly Asp Val Ala Ala Leu Arg Gly Ser Arg Lys Phe
    370                 375                 380
Asn Ile Leu Gly Thr Asn Thr Lys Val Met Asn Met Glu Glu Ser Asn
385                 390                 395                 400
Asn Gly Ser Leu Ser Ala Glu Phe Lys His Leu Thr Leu Arg Glu Gln
                405                 410                 415
Arg Cys Gly Asn Gly Gly Arg Ala Asn Cys Asp Ala Ser Leu Ile Val
            420                 425                 430
Thr Glu Glu Leu His Leu Ile Thr Phe Glu Thr Glu Val Tyr His Gln
        435                 440                 445
Gly Leu Lys Ile Asp Leu Glu Thr His Ser Leu Pro Val Val Val Ile
    450                 455                 460
Ser Asn Ile Cys Gln Met Pro Asn Ala Trp Ala Ser Ile Leu Trp Tyr
465                 470                 475                 480
Asn Met Leu Thr Asn Asn Pro Lys Asn Val Asn Phe Phe Thr Lys Pro
                485                 490                 495
Pro Ile Gly Thr Trp Asp Gln Val Ala Glu Val Leu Ser Trp Gln Phe
            500                 505                 510
Ser Ser Thr Thr Lys Arg Gly Leu Ser Ile Glu Gln Leu Thr Thr Leu
        515                 520                 525
Ala Glu Lys Leu Leu Gly Pro Gly Val Asn Tyr Ser Gly Cys Gln Ile
    530                 535                 540
Thr Trp Ala Lys Phe Cys Lys Glu Asn Met Ala Gly Lys Gly Phe Ser
545                 550                 555                 560
Phe Trp Val Trp Leu Asp Asn Ile Ile Asp Leu Val Lys Lys Tyr Ile
                565                 570                 575
Leu Ala Leu Trp Asn Glu Gly Tyr Ile Met Gly Phe Ile Ser Lys Glu
            580                 585                 590
Arg Glu Arg Ala Ile Leu Ser Thr Lys Pro Pro Gly Thr Phe Leu Leu
        595                 600                 605
Arg Phe Ser Glu Ser Ser Lys Glu Gly Gly Val Thr Phe Thr Trp Val
    610                 615                 620
Glu Lys Asp Ile Ser Gly Lys Thr Gln Ile Gln Ser Val Glu Pro Tyr
625                 630                 635                 640
Thr Lys Gln Gln Leu Asn Asn Met Ser Phe Ala Glu Ile Ile Met Gly
                645                 650                 655
Tyr Lys Ile Met Asp Ala Thr Asn Ile Leu Val Ser Pro Leu Val Tyr

                660                 665                 670
Leu Tyr Pro Asp Ile Pro Lys Glu Glu Ala Phe Gly Lys Tyr Cys Arg
        675                 680                 685
Pro Glu Ser Gln Glu His Pro Glu Ala Asp Pro Gly Ser Ala Ala Pro
    690                 695                 700
Tyr Leu Lys Thr Lys Phe Ile Cys Val Thr Pro Thr Thr Cys Ser Asn
705                 710                 715                 720
Thr Ile Asp Leu Pro Met Ser Pro Arg Thr Leu Asp Ser Leu Met Gln
                725                 730                 735
Phe Gly Asn Asn Gly Glu Gly Ala Glu Pro Ser Ala Gly Gly Gln Phe
            740                 745                 750
Glu Ser Leu Thr Phe Asp Met Asp Leu Thr Ser Glu Cys Ala Thr Ser
        755                 760                 765
Pro Met
    770

<210> 3
<211> 770
<212> PRT
<213> Rattus norvegicus

<400> 3
Met Ala Gln Trp Asn Gln Leu Gln Gln Leu Asp Thr Arg Tyr Leu Glu
1               5                   10                  15
Gln Leu His Gln Leu Tyr Ser Asp Ser Phe Pro Met Glu Leu Arg Gln
            20                  25                  30
Phe Leu Ala Pro Trp Ile Glu Ser Gln Asp Trp Ala Tyr Ala Ala Ser
        35                  40                  45
Lys Glu Ser His Ala Thr Leu Val Phe His Asn Leu Leu Gly Glu Ile
    50                  55                  60
Asp Gln Gln Tyr Ser Arg Phe Leu Gln Glu Ser Asn Val Leu Tyr Gln
65                  70                  75                  80
His Asn Leu Arg Arg Ile Lys Gln Phe Leu Gln Ser Arg Tyr Leu Glu
                85                  90                  95
Lys Pro Met Glu Ile Ala Arg Ile Val Ala Arg Cys Leu Trp Glu Glu
            100                 105                 110
Ser Arg Leu Leu Gln Thr Ala Ala Thr Ala Ala Gln Gln Gly Gly Gln
        115                 120                 125
Ala Asn His Pro Thr Ala Ala Val Val Thr Glu Lys Gln Gln Met Leu
    130                 135                 140
Glu Gln His Leu Gln Asp Val Arg Lys Arg Val Gln Asp Leu Glu Gln
145                 150                 155                 160
Lys Met Lys Val Val Glu Asn Leu Gln Asp Asp Phe Asp Phe Asn Tyr
                165                 170                 175
Lys Thr Leu Lys Ser Gln Gly Asp Met Gln Asp Leu Asn Gly Asn Asn
            180                 185                 190
Gln Ser Val Thr Arg Gln Lys Met Gln Gln Leu Glu Gln Met Leu Thr
        195                 200                 205
Ala Leu Asp Gln Met Arg Arg Ser Ile Val Ser Glu Leu Ala Gly Leu
    210                 215                 220
Leu Ser Ala Met Glu Tyr Val Gln Lys Thr Leu Thr Asp Glu Glu Leu
225                 230                 235                 240
Ala Asp Trp Lys Arg Arg Gln Gln Ile Ala Cys Ile Gly Gly Pro Pro
                245                 250                 255
Asn Ile Cys Leu Asp Arg Leu Glu Asn Trp Ile Thr Ser Leu Ala Glu
            260                 265                 270
Ser Gln Leu Gln Thr Arg Gln Gln Ile Lys Lys Leu Glu Glu Leu Gln
        275                 280                 285
Gln Lys Val Ser Tyr Lys Gly Asp Pro Ile Val Gln His Arg Pro Met
    290                 295                 300
Leu Glu Glu Arg Ile Val Asp Leu Phe Arg Asn Leu Met Lys Ser Ala
305                 310                 315                 320
Phe Val Val Glu Arg Gln Pro Cys Met Pro Met His Pro Asp Arg Pro
                325                 330                 335
Leu Val Ile Lys Thr Gly Val Gln Phe Thr Thr Lys Val Arg Leu Leu
            340                 345                 350
Val Lys Phe Pro Glu Leu Asn Tyr Gln Leu Lys Ile Lys Val Cys Ile
        355                 360                 365
Asp Lys Asp Ser Gly Asp Val Ala Ala Leu Arg Gly Ser Arg Lys Phe

370                 375                 380
Asn Ile Leu Gly Thr Asn Thr Lys Val Met Asn Met Glu Glu Ser Asn
385                 390                 395                 400
Asn Gly Ser Leu Ser Ala Glu Phe Lys His Leu Thr Leu Arg Glu Gln
                405                 410                 415
Arg Cys Gly Asn Gly Gly Arg Ala Asn Cys Asp Ala Ser Leu Ile Val
            420                 425                 430
Thr Glu Glu Leu His Leu Ile Thr Phe Glu Thr Glu Val Tyr His Gln
        435                 440                 445
Gly Leu Lys Ile Asp Leu Glu Thr His Ser Leu Pro Val Val Val Ile
    450                 455                 460
Ser Asn Ile Cys Gln Met Pro Asn Ala Trp Ala Ser Ile Leu Trp Tyr
465                 470                 475                 480
Asn Met Leu Thr Asn Asn Pro Lys Asn Val Asn Phe Phe Thr Lys Pro
                485                 490                 495
Pro Ile Gly Thr Trp Asp Gln Val Ala Glu Val Leu Ser Trp Gln Phe
            500                 505                 510
Ser Ser Thr Thr Lys Arg Gly Leu Ser Ile Glu Gln Leu Thr Thr Leu
        515                 520                 525
Ala Glu Lys Leu Leu Gly Pro Gly Val Asn Tyr Ser Gly Cys Gln Ile
    530                 535                 540
Thr Trp Ala Lys Phe Cys Lys Glu Asn Met Ala Gly Lys Gly Phe Ser
545                 550                 555                 560
Phe Trp Val Trp Leu Asp Asn Ile Ile Asp Leu Val Lys Lys Tyr Ile
                565                 570                 575
Leu Ala Leu Trp Asn Glu Gly Tyr Ile Met Gly Phe Ile Ser Lys Glu
            580                 585                 590
Arg Glu Arg Ala Ile Leu Ser Thr Lys Pro Pro Gly Thr Phe Leu Leu
        595                 600                 605
Arg Phe Ser Glu Ser Ser Lys Glu Gly Gly Val Thr Phe Thr Trp Val
    610                 615                 620
Glu Lys Asp Ile Ser Gly Lys Thr Gln Ile Gln Ser Val Glu Pro Tyr
625                 630                 635                 640
Thr Lys Gln Gln Leu Asn Asn Met Ser Phe Ala Glu Ile Ile Met Gly
                645                 650                 655
Tyr Lys Ile Met Asp Ala Thr Asn Ile Leu Val Ser Pro Leu Val Tyr
            660                 665                 670
Leu Tyr Pro Asp Ile Pro Lys Glu Glu Ala Phe Gly Lys Tyr Cys Arg
        675                 680                 685
Pro Glu Ser Gln Glu His Pro Glu Ala Asp Pro Gly Ser Ala Ala Pro
    690                 695                 700
Tyr Leu Lys Thr Lys Phe Ile Cys Val Thr Pro Thr Thr Cys Ser Asn
705                 710                 715                 720
Thr Ile Asp Leu Pro Met Ser Pro Arg Thr Leu Asp Ser Leu Met Gln
                725                 730                 735
Phe Gly Asn Asn Gly Glu Gly Ala Glu Pro Ser Ala Gly Gly Gln Phe
            740                 745                 750
Glu Ser Leu Thr Phe Asp Met Asp Leu Thr Ser Glu Cys Ala Thr Ser
        755                 760                 765
Pro Met
    770

<210> 4
<211> 186
<212> PRT
<213> Homo sapiens

<400> 4
   Met Glu Lys Lys Arg Arg Ala Arg Ile Asn Val Ser Leu Glu Gln Leu
   1               5                   10                  15
   Lys Ser Leu Leu Glu Lys His Tyr Ser His Gln Ile Arg Lys Arg Lys
               20                  25                  30
   Leu Glu Lys Ala Asp Ile Leu Glu Leu Ser Val Lys Tyr Met Arg Ser
           35                  40                  45
   Leu Gln Asn Ser Leu Gln Gly Leu Trp Pro Val Pro Arg Gly Ala Glu
       50                  55                  60
   Gln Pro Ser Gly Phe Arg Ser Cys Leu Pro Gly Val Ser Gln Leu Leu
   65                  70                  75                  80
   Arg Arg Gly Asp Glu Val Gly Ser Gly Leu Arg Cys Pro Leu Val Pro
   85 90 95
   Glu Ser Ala Ala Gly Ser Thr Met Asp Ser Ala Gly Leu Gly Gln Glu
   100 105 110
   Ala Pro Ala Leu Phe Arg Pro Cys Thr Pro Ala Val Trp Ala Pro Ala
   115 120 125
   Pro Ala Ala Gly Gly Pro Arg Ser Pro Pro Pro Leu Leu Leu Leu Pro
   130 135 140
   Glu Ser Leu Pro Gly Ser Ser Ala Ser Val Pro Pro Pro Gln Pro Ala
   145 150 155 160
   Ser Ser Arg Cys Ala Glu Ser Pro Gly Leu Gly Leu Arg Val Trp Arg
   165 170 175
   Pro Trp Gly Ser Pro Gly Asp Asp Leu Asn
   180 185

<210> 5
<211> 175
<212> PRT
<213> Mus musculus

<400> 5
Met Glu Lys Lys Arg Arg Ala Arg Ile Asn Val Ser Leu Glu Gln Leu
1               5                   10                  15
Arg Ser Leu Leu Glu Arg His Tyr Ser His Gln Ile Arg Lys Arg Lys
            20                  25                  30
Leu Glu Lys Ala Asp Ile Leu Glu Leu Ser Val Lys Tyr Met Arg Ser
        35                  40                  45
Leu Gln Asn Ser Leu Gln Gly Leu Trp Pro Val Pro Ser Gly Val Asp
    50                  55                  60
Tyr Pro Ser Gly Phe Gln Gly Gly Leu Arg Gly Val Ser Gln Arg Leu
65                  70                  75                  80
Arg Pro Gly Glu Gly Asp Ser Gly Leu Arg Cys Pro Leu Leu Leu Gln
                85                  90                  95
Arg Arg Glu Gly Ser Thr Thr Asp Ser Ala Asn Pro Gln Ala Thr Ser
            100                 105                 110
Val Leu Asn Pro Cys Leu Pro Ala Ile Trp Ala Pro Ser Arg Ala Ala
        115                 120                 125
Gly Gly Ser His Ser Pro Gln Ser Pro Leu Pro Leu Pro Gly Gly Leu
    130                 135                 140
Leu Glu Ser Ser Thr Asp Val Val Ala Pro His Pro Ala Ser Asn Cys
145                 150                 155                 160
Gln Ala Glu Ser Thr Arg Pro Gly Phe Arg Val Trp Arg Pro Trp
                165                 170                 175

<210> 6
<211> 200
<212> PRT
<213> Mus musculus

<400> 6
Met Gly Thr Glu Pro Thr Ser Arg Asp Ser Leu Gly Gly Pro Ala Ser
1               5                   10                  15
Ser Phe Arg Lys Ile Ser Lys Pro Leu Met Glu Lys Lys Arg Arg Ala
            20                  25                  30
Arg Ile Asn Val Ser Leu Glu Gln Leu Arg Ser Leu Leu Glu Arg His
        35                  40                  45
Tyr Ser His Gln Ile Arg Lys Arg Lys Leu Glu Lys Ala Asp Ile Leu
    50                  55                  60
Glu Leu Ser Val Lys Tyr Met Arg Ser Leu Gln Asn Ser Leu Gln Gly
65                  70                  75                  80
Leu Trp Pro Val Pro Ser Gly Val Asp Tyr Pro Ser Gly Phe His Gly
                85                  90                  95
Gly Leu Arg Gly Val Ser Gln Arg Leu Arg Pro Gly Glu Gly Asp Ser
            100                 105                 110
Gly Leu Arg Cys Pro Leu Leu Leu Gln Arg Arg Glu Gly Ser Thr Thr
        115                 120                 125
Asp Ser Ala Asn Pro Gln Ala Thr Ser Val Leu Asn Pro Cys Leu Pro
    130                 135                 140
Ala Ile Trp Ala Pro Ser Arg Ala Ala Gly Gly Ser His Ser Pro Gln
145                 150                 155                 160

Ser Pro Leu Pro Leu Pro Gly Gly Leu Leu Glu Ser Ser Thr Asp Val
                165                 170                 175
Val Ala Pro Gln Pro Ala Ser Asn Cys Gln Ala Glu Ser Thr Arg Pro
            180                 185                 190
Gly Phe Arg Val Trp Arg Pro Trp
        195                 200

<210> 7
<211> 175
<212> PRT
<213> Rattus norvegicus

<400> 7
Met Glu Lys Lys Arg Arg Ala Arg Ile Asn Leu Ser Leu Glu Gln Leu
1               5                   10                  15
Arg Ser Leu Leu Glu Arg His Tyr Ser His Gln Ile Arg Lys Arg Lys
            20                  25                  30
Leu Glu Lys Ala Asp Ile Leu Glu Leu Ser Val Lys Tyr Val Arg Ser
        35                  40                  45
Leu Gln Asn Ser Leu Gln Gly Leu Trp Leu Val Pro Ser Gly Val Asp
    50                  55                  60
Tyr Pro Ser Gly Phe Arg Gly Gly Leu Pro Gly Ser Ser Gln Arg Leu
65                  70                  75                  80
Arg Pro Gly Glu Asp Asp Ser Gly Leu Arg Cys Pro Leu Leu Leu Gln
                85                  90                  95
Arg Arg Ala Gly Ser Thr Thr Asp Ser Ala Asn Pro Gln Thr Ala Ser
            100                 105                 110
Val Leu Ser Pro Cys Leu Pro Ala Ile Trp Ala Pro Gly Pro Pro Ala
        115                 120                 125
Gly Gly Ser Gln Ser Pro Gln Ser Pro Phe Pro Pro Leu Gly Gly Leu
    130                 135                 140
Leu Glu Ser Ser Thr Gly Ile Leu Ala Pro Pro Pro Ala Ser Asn Cys
145                 150                 155                 160
Gln Ala Glu Asn Pro Arg Pro Gly Phe Arg Val Trp Arg Pro Trp
                165                 170                 175

<210> 8
<211> 697
<212> DNA
<213> Homo sapiens

<400> 8
tgggcacgga gcctgctgcc cggggcagcc tccccggcaa cttccgaaag atttccaagc      60

cgctgatgga gaaaaagcgc cgggcacgca tcaatgtgtc actggagcag ctcaagtcgc     120

tgctggagaa acactactcg caccagatcc ggaagcgcaa attggagaag gccgacatcc     180

tggagttgag cgtgaagtac atgagaagcc ttcagaactc cttgcaaggg ctctggcctg     240

tgcccagggg agccgagcaa ccgtcgggct tccgcagctg cctgcccggc gtgagccagc     300

tccttcggcg cggagatgag gtcggcagcg gcctgcgctg ccccctggtg cccgagagcg     360

ccgccggcag caccatggac agcgccgggt tgggccagga ggcgcccgcg ctgttccgcc     420

cttgcacccc tgccgtctgg gctcctgctc cggccgccgg cggcccgcgg tccccaccac     480

ccctgctcct cctccccgaa agtctccctg gctcgtccgc cagcgtcccc ccgccgcagc     540

cagcgtcgag tcgctgcgcc gagagtcccg ggctgggcct gcgcgtgtgg cggccctggg     600

gaagccccgg ggatgacctg aactgaaggc gctccctatt tggtctcgcg acacagggac     660

tattttcagc acgcccacag tgactgccag gacccccc                             697

<210> 9
<211> 1829

<212> DNA
<213> Mus musculus

<400> 9
gctcagagag gacaaagtga gcagctgtct ctccttaccg ctgggaaagc tctcttctgg 60

gtgggtctgc ctgtggacgc aggccctgct tgcgactggc tttgagcaac cacaggcaga 120

aaattgctac attgtcctga cattagggcg actaattcag catcccagcg tggccagagc 180

gtgaagggag cagaaaagca tcactggcca gcatccttgc ctcagcatct ttatcaggcc 240

tcaggacacc cagagctcca ccatcatcat ttaggacggc ccccagctct caaagctccc 300

tgccatagcg gaggaccacc agaagaatgc tggctaagaa gcagccagga cttggcaagc 360

agacatagtg atctccaagc ctctgatgga gaagaagcgc cgtgcacgca tcaacgtgtc 420

actggagcag ctgaggtctc ttctggagag acactactca catcagatac ggaaacgaaa 480

gctggagaag gccgatatcc tggagctgag tgttaagtac atgagaagcc tccagaactc 540

actgcaagga ctctggccag tacccagtgg ggtggactac ccgtccggct tccaaggcgg 600

cttgcgtggc gtgagccaga ggcttcggcc cggagaggga gacagcggcc tgcgctgccc 660

cttgctgctc cagcgcaggg aaggcagcac cacggacagc gccaacccac aggcgacctc 720

tgttctcaac ccttgcctcc cggccatctg ggcccccagt cgcgctgcag gtggctccca 780

ctccccgcag tccccgctcc ctctccctgg aggtctcctt gagtcttcca ccgatgttgt 840

ggcaccacat cctgcatcaa actgccaggc cgagagtacg agacccgggt ttcgcgtgtg 900

gcgaccgtgg tgagacgtag gcaaggattt agactcaagc tctctcccca cgtccccatc 960

accccgccat ctagtctggg gaaactattt tggggcaccc atggtgactg caggaactct 1020

atcaatcggt ggcctggggg gggggagtgc aggaaatcca cggaaccatt ggcgattcgg 1080

gtctgcactg ctaggtgaac tcatccactc cagccccacc cactccatcc agaccagctc 1140

tctacctccc gcttctagag aagatgggag gggacgggtg agctgtaccc ctttcccagg 1200

gacccggggc tggcagcccg cccctgcccc ctctccacgg ctgcgtcacg gtgagaaggt 1260

cgcggttgct tgcagcacag agcggacccc tcgcagcgag gagaatcgct gccccgccct 1320

ggcccattca accggcctcg cgcgctccgt tcccaccggc acaaagcggg ccgggctccg 1380

ccccttcagc aaggggctcc catcctatca ccacccaaga ccttctcact accatcgaaa 1440

cccatcaccc cctctacacc gattccacac tgctggggcg ctctcaggcc ctcaactgcg 1500

caaatactta tggctgcggc gtgctggcgt agaacattgg atccgcccct aaggtacttg 1560

cgtgcccgac gccccgccta gccacaatct atagtcctgg gtcccaccca tccctgctta 1620

gcactgctga gacctgagca ttttgtgttc tgcaaagacc actttagtgc cttctgagcc 1680

ctggactcga actaagagcc tgttagttac catgagcgtc gactgtgttg gggagagggc 1740

tcctgtcgtg gaccagtctg gggtccagtg caaaatccca gaatctctta ttaccaatat 1800

acaattatga ataaagactg ggcgtgaat 1829

<210> 10
<211> 964

<212> DNA
<213> Rattus norvegicus

<400> 10
   gtggatgcag gccctgcttg caactggctt tgagcaacca caggcagaaa attgctacat 60
   tgtcctgaca ttagggcgac taattcagca tcccagcgtg gccagagcgt gaagggagca 120
   gaaaagcatc actggccagc atcctcgcct cagcatcttt atcaggcctc aggacaccta 180
   gcgccccacc atcatcattt aggatggacc ccagctcaca cagctccctg ccataacgga 240
   ggaccaccag aagaatgttg gatctccaag cctctgatgg agaagaagcg tcgtgcccgc 300
   atcaacctgt cactggagca gctaaggtct cttctggaga gacactactc ccaccagata 360
   cggaagcgaa agctggagaa ggccgatatc ctggagctga gcgttaagta cgtgagaagc 420
   ctccagaact cattgcaagg actttggcta gtacccagtg gggtggacta cccgtccgga 480
   ttccgaggcg gcttgccggg ctccagccag aggcttcggc ccggagagga cgacagcggc 540
   ctgcgctgcc ccctgcttct ccagcgcagg gcaggcagca ccacggacag cgccaaccca 600
   cagacggcct ctgttctcag cccctgcctc ccggccatct gggcccctgg tccccctgca 660
   ggtggctccc aatccccgca gtccccgttc cctccccttg gaggtctcct tgagtcctcc 720
   actggcattc tggcaccgcc acctgcatca aactgccagg ccgagaatcc gagacccggg 780
   tttcgcgtgt ggcggccctg gtgaggccta ggcaagaatt tagattcaag ccctaacctc 840
   tgcccttatc acctcgccgt ctagtttggg ggaaactatt tggggacacc catggtgact 900
   gcagggactc tatcaatcgg tggcctggtg gggctgcagg gaaatacacg gagccatttg 960
   ctga 964